# EUROPEAN PATENT SPECIFICATION

(11) **EP 3 419 576 B1**
(45) Date of publication and mention of the grant of the patent: **19.08.2020**
(21) Application number: 17709275.6
(22) Date of filing: 23.02.2017
(51) Int. Cl.: A61F 13/42, A61F 13/45, A61F 13/475, A61F 13/511, A61F 13/495

(54) **SAUGFÄHIGE WICKELUNTERLAGEN**
SAUGFÄHIGE WICKELUNTERLAGEN
MATELAS À LANGER JETABLES ABSORBANTS

(30) Priority: 25.02.2016 US 201662299733 P; 17.06.2016 US 201662351327 P; 11.08.2016 US 201615234235
(43) Date of publication of application: 02.01.2019
(73) Proprietor: The Procter & Gamble Company, Cincinnati, OH 45202 (US)
(72) Inventor: LUDWIG, Susan, Joy, Cincinnati Ohio 45202 (US); TALLY, Amy, Lynn, Cincinnati Ohio 45202 (US); MORRIS, Taylor, Javier, Cincinnati Ohio 45202 (US); ROE, Donald, Carroll, Cincinnati Ohio 45202 (US)
(74) Representative: P&G Patent Germany
(86) International application number: PCT/US2017/019045
(87) International publication number: WO 2017/147244

(56) References cited:
- EP-A1- 1 064 899
- WO-A1-94/14395
- WO-A1-99/33422
- WO-A1-2006/123973
- US-A1- 2014 221 956
- US-A1- 2015 282 997

## Description

### FIELD

The present disclosure is directed to absorbent pads, and is more specifically directed to absorbent pads that are configured to be placed on a surface to absorb bodily exudates from babies positioned on the absorbent pads.

### BACKGROUND

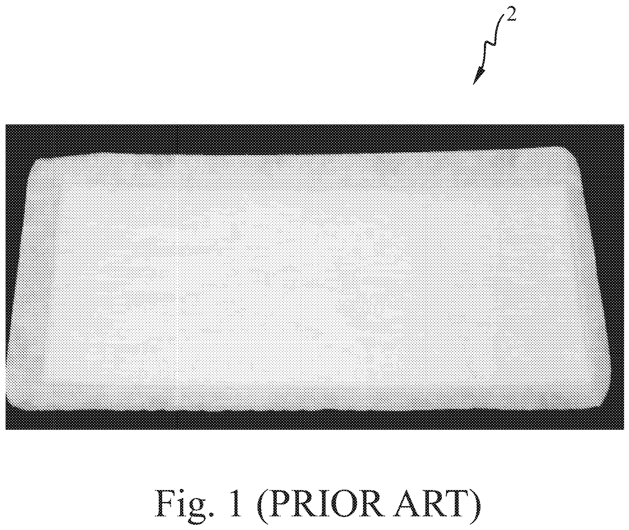

Absorbent pads are used to contain bodily exudates (e.g., urine and BM) from babies, including premature babies and Neonatal Abstinence Syndrome ("NAS") babies. These absorbent pads are typically placed on a surface and then the babies are laid on top of them. Males are typically placed face-down and females may be placed either face-down or face-up. Fig. 1 illustrates an example of a prior art absorbent pad 2. The absorbent pad 2 may include a topsheet, a backsheet, and an absorbent core positioned between the topsheet and the backsheet.

Some of the babies placed on these absorbent pads require special care by nurses and other hospital staff, especially premature and NAS babies. These babies are oftentimes on ventilators, feeding tubes, or other life support systems. In some instances, the babies may be positioned within incubators, also known as isolets. It is desirable to keep these babies in high humidity, very sterile environments. Furthermore, the babies have very delicate skin that needs to be protected. Current absorbent pads for babies do not provide adequate containment of bodily exudates and skin protection from the bodily exudates. As such, absorbent pads for babies need to be improved.

WO99/33422A1 discloses male personal hygiene articles comprising a moisture barrier, a liner bonded to the moisture barrier and an absorbent assembly sandwiched therebetween. The articles comprise an inner pair of elastics extending the full length of the article that causes the article to take a three-dimensional U shape when positioned on a flat surface, incompatible with having a baby lying on it. WO99/33422A1 does not disclose a non-slip means on a surface of the absorbent pad opposite to the topsheet.

### SUMMARY

The present disclosure provides absorbent pads configured to be positioned on a flat, horizontal surface to absorb bodily fluids exudates from babies positioned on the absorbent pad that overcome the disadvantages of the prior art by providing better bodily exudate containment and skin protection from the bodily exudates. The absorbent pads of the present disclosure comprises a single pair of cuffs or raised barriers that help contain bodily exudates over an absorbent core so that they can be absorbed into the absorbent core. The cuffs or raised barriers may also help contain the bodily exudates within the absorbent pad and prevent, or at least inhibit, bodily exudate leakage out the sides and/or ends of the absorbent pads. In the prior art absorbent pads, bodily exudates may wick out the sides and/or ends of the absorbent pad, thereby causing undesirable environments for the babies and their caregivers.

The absorbent pad comprises a central longitudinal axis, a central lateral axis, a first end edge on a first side of the central lateral axis, a second end edge on a second side of the central lateral axis, a first side edge on a first side of the central longitudinal axis, and a second side edge on a second side of the central longitudinal axis. The absorbent pad comprises a liquid permeable, apertured, or non-apertured, topsheet and a liquid permeable backsheet. The topsheet may be hydrophobic or hydrophilic, or have hydrophobic or hydrophilic portions or layers. The absorbent pad comprises an absorbent core disposed at least partially intermediate the liquid permeable topsheet and the liquid impermeable backsheet. The absorbent core may be rectangular or shaped. The absorbent core may have notches cut out of it proximate to or overlapping the central lateral axis to allow the absorbent core to be folded easier. The absorbent pad comprises a single pair of cuffs extending at least partially intermediate the first end edge and the second end edge. The cuffs comprise an elastic. Each elastic, may be joined to the cuff only in a joined area or a longitudinally central joined area. Portions of the elastics outside of the joined areas are free from attachment to the cuffs.

The absorbent pad comprises a first wetness guard positioned on the first side of the central lateral axis and having a portion positioned proximate to the first end edge. The first wetness guard is positioned over at least a first portion of the topsheet and crosses the central longitudinal axis. The first wetness guard comprises a first nonwoven material and a first liquid impermeable film. The first wetness guard forms a first portion of a wearer-facing surface of the absorbent pad. The absorbent pad comprises a second wetness guard positioned on the second side of the central longitudinal axis and having a portion positioned proximate to the second end edge. The second wetness guard is positioned over at least a second portion of the topsheet and crosses the central longitudinal axis. The second wetness guard comprises a second nonwoven material and a second liquid impermeable film. The second wetness guard forms a second portion of the wearer-facing surface.

The wetness guards are positioned in areas of the absorbent pads that come into contact with a baby's back, chest, or leg, but not be positioned where a crotch area of the baby contacts the absorbent pad. The non-film material may face toward the baby, while the liquid impermeable material may face away from the baby. The wetness guards may accomplish at least two functions. First, when a baby urinates, the urine is typically wicked away from the point of entry into the absorbent core throughout the absorbent core. This may cause areas of the absorbent pad where the baby's back, chest, and/or legs are to be wet and unsterile. The wetness guards provide a barrier between the wet and unsterile areas and the baby's back, chest, and/or legs, thereby leading to improved skin health for the baby. Second, the wetness guards may comprise soft non-film materials facing the baby's skin. As such, the baby's chest, back, and/or legs may rest on a soft, comfortable material again leading to improved skin health.

The absorbent pads of the invention are further defined in the attached claims.

### BRIEF DESCRIPTION OF THE DRAWINGS

While the specification concludes with claims particularly pointing out and distinctly claiming the subject matter which is regarded as forming the present invention, it is believed that the present disclosure will be better understood from the following description which is taken in conjunction with the accompanying drawings in which the designations are used to designate substantially identical elements and in which:
Fig. 1 is a photograph of a prior art absorbent pad;
Fig. 2 is a photograph of an example absorbent pad of the present disclosure;
Fig. 3 is a schematic illustration of a front view of an absorbent pad of the present disclosure, wearer-facing surface facing the viewer;
Fig. 4 is a schematic illustration of another absorbent pad of the present disclosure, wearer-facing surface facing the viewer, which is not part of the claimed invention;
Fig. 5 is a schematic illustration of a back view of the absorbent pad of Fig. 4;
Fig. 6 is a photograph of a portion of the absorbent pad of Fig. 2;
Fig. 7 is a schematic cross-sectional illustration of the absorbent pad of Fig. 3, taken about line 7-7 of Fig. 3;
Fig. 7A is a cross-sectional illustration of a cuff of Fig. 7 that is free of elastics;
Fig. 8 is a schematic illustration of a front view of another absorbent pad of the present disclosure, wearer-facing surface facing the viewer, which is not part of the claimed invention;
Fig. 9 is a schematic illustration of a front view of another absorbent pad of the present disclosure, wearer-facing surface facing the viewer;
Fig. 10 is a schematic illustration of a front view of another absorbent pad of the present disclosure, wearer-facing surface facing the viewer;
Fig. 11A is a schematic illustration of a front view of another absorbent pad of the present disclosure, wearer-facing surface facing the viewer;
Fig. 11B is a schematic illustration of a back view of a discrete wetness guard according to the present disclosure, non-wearer-facing surface facing the viewer;
Fig. 12 is a schematic illustration of a front view of another absorbent pad comprising a sleeve-type wetness guard of the present disclosure, wearer-facing surface facing the viewer;
Fig. 13 is a schematic cross-sectional example illustration of the sleeve-type wetness guard of Fig. 12;
Fig. 14 is a schematic illustration of a front view of another absorbent pad comprising leakage guards of the present disclosure, wearer-facing surface facing the viewer;
Fig. 15 is a schematic illustration of a front view of another absorbent pad comprising leakage guards of the present disclosure, wearer-facing surface facing the viewer;
Figs. 16-18 are schematic illustrations of absorbent pads showing graphics and/or indicia, garment-facing surfaces facing the viewer;
Fig. 19 is a plan view of an example of a discrete fastening member for an absorbent pad of the present disclosure, garment-facing surface facing the viewer;
Fig. 20 is a plan view of an example of a discrete fastening member for an absorbent pad of the present disclosure, wearer-facing surface facing the viewer;
Fig. 21 is a cross-sectional view of the discrete fastening member taken about line 21-21 of Fig. 20;
Fig. 22 is a plan view of an example of a discrete fastening member for an absorbent article of the present disclosure, garment-facing surface facing the viewer;
Fig. 23 is a plan view of an example of a discrete fastening member for an absorbent article of the present disclosure, wearer-facing surface facing the viewer;
Fig. 24 is a plan view of an example absorbent pad of the present disclosure, wearer-facing surface facing the viewer;
Fig. 25 is a photograph of two absorbent pads of the present disclosure, the top absorbent pad having a joined area in cuffs and the bottom absorbent pad not having a joined area in the cuffs;
Fig. 26 is a plan view of an example absorbent pad of the present disclosure, wearer-facing surface facing the viewer;
Fig. 27 is a perspective side view of a curled or folded over portion of an absorbent pad.
Fig. 28 is another perspective view of the curled or folded over portion of the absorbent pad of Fig. 28;
Fig. 29 is a plan view of an example absorbent pad of the present disclosure, wearer-facing surface facing the viewer;
Fig. 30 is a plan view of an example absorbent pad of the present disclosure, wearer-facing surface facing the viewer;
Fig. 31 is a perspective view photograph of an absorbent pad with folded over cuffs, wearer-facing surface facing away from the surface on which the absorbent pad is resting;
Fig. 32 is a plan view of an absorbent pad with folded over cuffs;
Fig. 33 is an example cross-sectional view of the absorbent pad prior to the cuffs being folded toward the longitudinal axis;
Fig. 34 is an example end view of the absorbent pad of Fig. 32 before any tack down bonds are applied;
Fig. 35 is an example plan view of an absorbent core of an absorbent pad; and
Fig. 36 is an example plan view of an absorbent core of an absorbent pad.

### DETAILED DESCRIPTION

Various non-limiting forms of the present disclosure will now be described to provide an overall understanding of the principles of the structure, function, manufacture, and use of the absorbent pads disclosed herein. One or more examples of these non-limiting forms are illustrated in the accompanying drawings. Those of ordinary skill in the art will understand that the absorbent pads specifically described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

As used herein, the terms "join" and "joined" encompass configurations whereby an element is directly secured to another element by affixing the element directly to the other element, and configurations whereby an element is indirectly secured to another element by affixing the element to intermediate member(s) which in turn are affixed to the other element.

As used herein, the term "array" means a display of packages comprising disposable articles (e.g., diapers and/or absorbent pads) of different sizes having like article constructions. In some instances, diapers and absorbent pads may be included in the same array or in different arrays. Packages for the products have the same brand and/or sub-brand, and the packages are oriented in proximity to each other in a given area of a retail store. An array is marketed as a line-up of products normally having like packaging elements (e.g., packaging material type, film, paper, dominant color, design theme, etc.) that conveys to consumers that the different individual packages are part of a larger line-up. Arrays often have the same brand, for example, "Pampers®." In other instances, the arrays may have brands from the same manufacturer, such as "Pampers®" and "Luvs®", for example.

Arrays also often have the same trademarks, including trademarks of the brand, sub-brand, and/or features and/or benefits across a line-up.

As used herein, the term "on-line array" means an "array" distributed by a common on-line source.

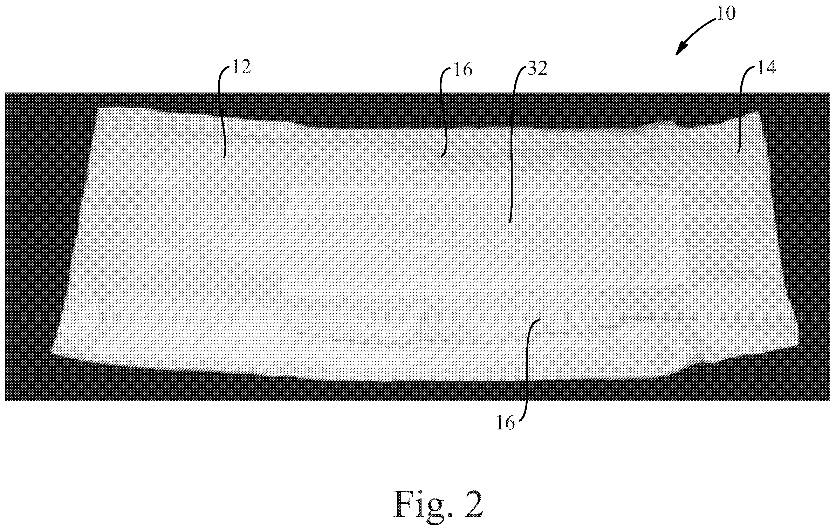

Absorbent pads that provide improved bodily exudate containment and improved skin health for babies are provided herein. The improved bodily exudate containment may be provided by cuffs or raised barriers on the absorbent pad, while the wetness guards may promote improved skin health for babies. The absorbent pads are configured to be positioned on a surface and then a baby can be laid on the absorbent pads. Referring to Fig. 2, a photograph of an example absorbent pad 10 of the present disclosure is illustrated. The absorbent pad 10 has a first wetness guard 12 and a second wetness guard 14. The absorbent pad 10 also comprises cuffs 16 or raised barriers for containing bodily exudates so that they may be absorbed by an absorbent core and remain within an area of the absorbent pad 10. This feature may reduce side leakage of the absorbent pads. In some instances, the absorbent pad may comprise an apertured topsheet to aid in the absorption of bodily exudates.

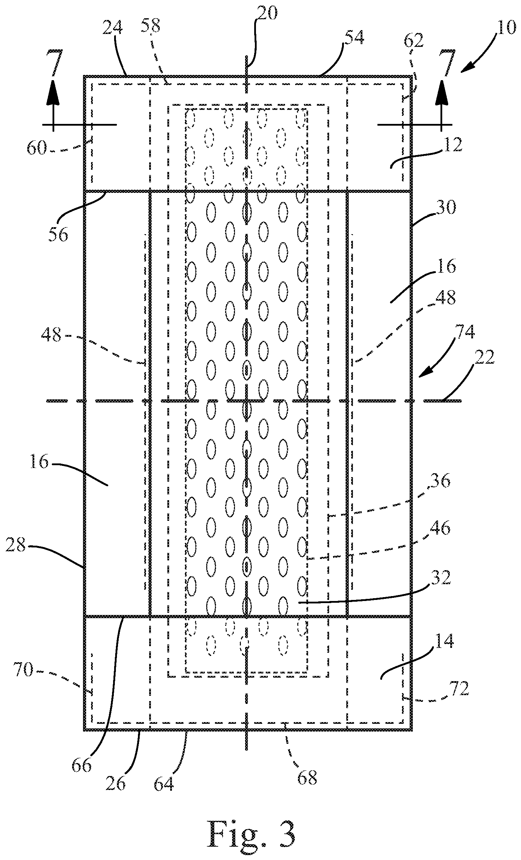

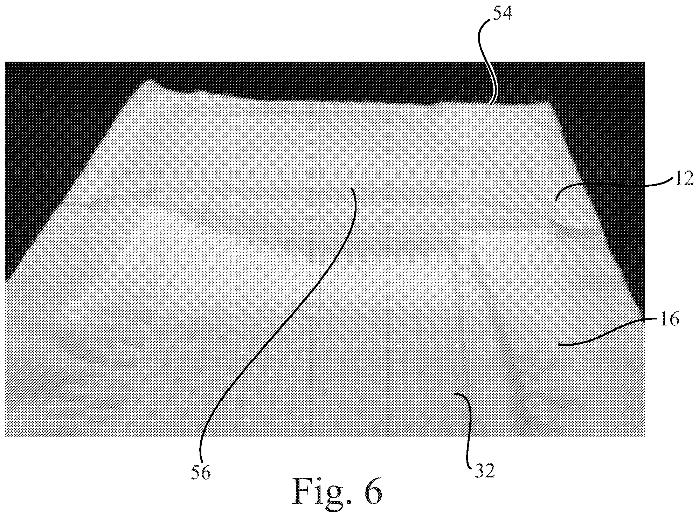

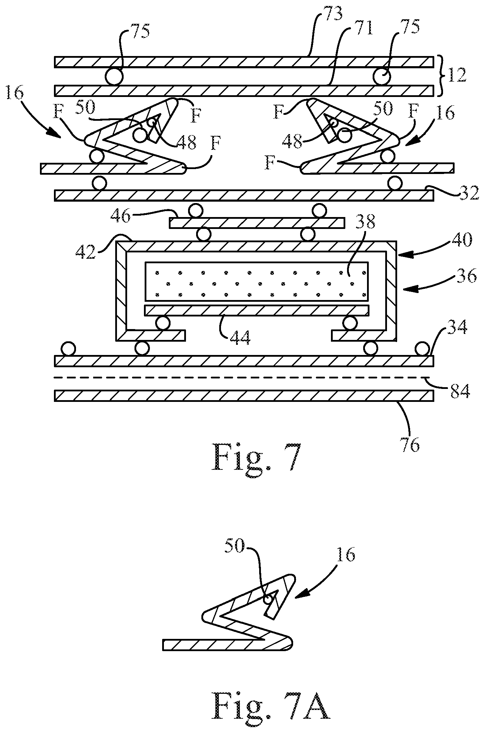

Fig. 3 is a schematic illustrations of front views of absorbent pads 10 of the present disclosure, wearer-facing surface facing the viewer. Fig. 6 is a photograph of a portion of the absorbent pad 10 of Fig. 2. Fig. 7 is a schematic cross-sectional illustration of the absorbent pad 10 of Fig. 3, taken about line 7-7. Fig. 7A is a cross-sectional illustration of a cuff of Fig. 7 that is free of elastics. Referring to Figs. 2-5, the absorbent pad 10 comprises a central longitudinal axis 20 and a central lateral axis 22. The absorbent pad 10 comprises a first end edge 24 on a first side of the central lateral axis 22 and a second end edge 26 on a second side of the central lateral axis 22. The absorbent pad 10 comprises a first side edge 28 on a first side of the central longitudinal axis 20 and a second side edge 30 on a second side of the central longitudinal axis 20. The absorbent pad 10 comprises a liquid permeable material or liquid permeable topsheet 32, a liquid impermeable material or liquid impermeable backsheet 34, and an absorbent core 36 positioned at least partially intermediate the topsheet 32 and the backsheet 34.

The topsheet 32, the backsheet 34, and the absorbent core 36 may be manufactured of any suitable materials. Suitable topsheet materials may comprise porous foams, reticulated foams, apertured plastic films, or woven or nonwoven webs of natural fibers (e.g., wood or cotton fibers), synthetic fibers (e.g., polyester or polypropylene fibers), non-apertured materials, apertured materials, apertured nonwoven materials, apertured nonwoven materials that are produced using an overbonding and ring rolling process, or materials having a combination of natural and synthetic fibers. Spunbond high loft materials may also be used, whether apertured or non-apertured. The topsheet 32 may have an embossed pattern, graphics, patterned, indicia, and/or three-dimensional features, either along with or instead of apertures. In some instances, the topsheet 32 may be a planar topsheet. In other instances, the topsheet may be a topsheet like that disclosed in U. S. Patent Application Publication No. 2015/0250662, to Isele et al., filed on March 2, 2015 or in U.S. Patent Application Publication No. 2016/0136014, to Arora et al., filed on November 5, 2015. The topsheet 32 may be hydrophobic or hydrophilic. If the topsheet is apertured, it may be desirable to have the topsheet be hydrophobic.

Suitable backsheet materials may comprise breathable materials that permit vapors to escape from the absorbent pad 10 while still preventing, or at least inhibiting, bodily exudates from passing through the backsheet 34 (i.e., liquid impermeable materials). Such materials may comprise nonwoven materials, woven materials, films, and/or laminates comprising a combination of one or more of these materials. Other backsheet materials may comprise non-breathable materials, such as films, for example.

A suitable absorbent core 36 for use in the absorbent pads 10 of the present disclosure may comprise any absorbent material which is generally compressible, conformable, non-irritating to the wearer's skin, and capable of absorbing and retaining bodily exudates. In addition, the configuration and construction of the absorbent core 36 may also be varied (e.g., the absorbent core(s) or other absorbent structure(s) may have varying caliper zones, hydrophilic gradient(s), a superabsorbent gradient(s), or lower average density and lower average basis weight acquisition zones; or may comprise one or more layers or structures). In other instances, the configuration and construction of the absorbent core 36 may be uniform and homogeneous. In such an instance, the absorbent material may be uniform and homogeneous (i.e., not profiled). The absorbent core 36 has a rectangular shape.

Referring to Fig. 7, the absorbent core 36 of the absorbent pads 10 of the present disclosure may comprise an absorbent material 38. The absorbent material 38 may comprise superabsorbent polymers, air-felt (cellulosic material), co-form, foams (including but not limited to High Internal Phase Emulsion foams), or mixtures thereof, for example. In some forms the absorbent material 38 may comprise at least 40%, at least 50%, at least 75%, at least 80%, at least 85%, at least 90%, at least 95%, at least 97%, at least 98%, at least 99%, or 100% superabsorbent polymers. In other forms, the absorbent material 42 may only comprise air-felt and be free of any superabsorbent polymers. In other forms, the absorbent material 38 may comprise a mixture of superabsorbent polymers and air-felt (e.g., 30% to 70% air-felt and 30% to 70% superabsorbent polymers). The absorbent material 38 may be enclosed in a core bag 40. The core bag 40 may comprise a first substrate 42 at least partially on a first side of the absorbent material 38 and a second substrate 44 at least partially on a second side of the absorbent material 38. The first substrate 42 may form a C-wrap around portions of the absorbent material 38 and the second material 44 to enclose the absorbent material 38. In an instance, the absorbent material 38 may have an hourglass shape and the absorbent core 36 may be rectangular (i.e., the core bag 40 is rectangular and the absorbent material 38 has an hourglass shape). In other instances, the core wrap may have any suitable configuration known to those of skill in the art. In some forms, the absorbent core may comprise one or more absorbent material free areas or channels, or substantially absorbent material free areas (e.g., areas with substantially no superabsorbent polymers or air-felt). In this instance, the first substrate 42 may be joined, bonded, or glued to the second substrate 44 in these areas or channels. In other forms, channels may be embossed into the absorbent core 36. Some example channel and absorbent core configurations are described in further detail in U.S. Patent Nos. 8,979,815, 9,216,118, and 9,216,116.

In an instance, the absorbent material may have a different shape as the core bag. For example, the core bag may be rectangular and the absorbent material may be hourglass shaped.

Referring to Figs. 3-5 and 7, the absorbent pad 10 may comprise one or more acquisition materials 46. The absorbent pad 10 may also comprise one or more distribution materials either between the topsheet 32 and the acquisition material 46 or between the acquisition material 46 and core bag 40. The acquisition material 46 may be used to aid the topsheet 32 in acquiring bodily exudates and moving them into the absorbent core 36. The distribution material, if provided, may help the absorbent pad 10 distribute bodily exudates about the absorbent core 36. The acquisition material 46 and/or the distribution material may have an hourglass shape, a rectangular shape, or an extended hourglass shape, for example. As an example, the acquisition material may be a nonwoven material and the distribution material may be cross-linked cellulosic fibers, tissue, or another nonwoven material, for example. In some instances, only an acquisition material may be provided, only a distribution may be provided, or neither may be provided depending on the desired properties of a particular absorbent pad.

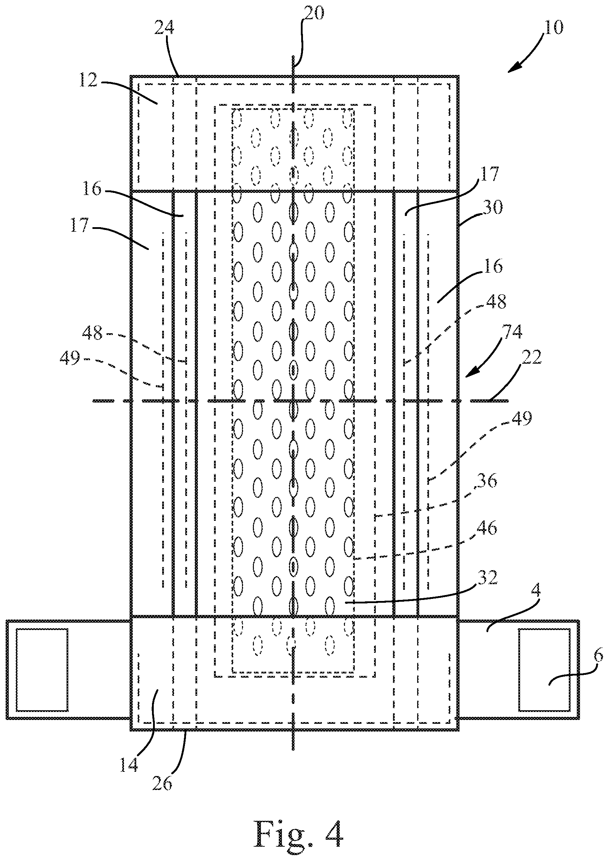

Referring to Figs. 2-7, the pair of cuffs 16 extends at least partially between, or fully between, the first end edge 24 and the second end edge 26. The cuffs 16 have one or more elastics 48 The absorbent pad 10 only has a single pair of cuffs 16 and is free of a second pair of cuffs as shown on Fig. 4 as a second pair of cuffs 17 The elastics 48 and 49 may cause the cuffs to stand (i.e., extend upwardly) relative to the topsheet 32. The elastics 48 and 49 may be any suitable length along the central longitudinal axis 20. In a form, the cuffs 16 (or 17) may each have three longitudinally extending folds (labeled F in Fig. 7) to allow them to stand and extend a suitable distance from the topsheet 32. Glue or bonding of the cuffs is indicated at 50 in Fig. 7. In other forms, the cuffs 16 or 17 may have any suitable configuration. The cuffs generally aid bodily exudate containment to the area of the absorbent core 36 and reduce side edge leakage. Contracted and/or standing portions of the cuffs 16 and/or 17 may be more prominent on a first side of the central lateral axis 22 or the second side of the central lateral axis 22. In other instances, the contracted and/or standing portions of the cuffs 16 and/or 17 may be the same on both sides of the central lateral axis 22. In some instances, the cuffs 16 and/or 17 may extend the same distance on each side of the central lateral axis 22.

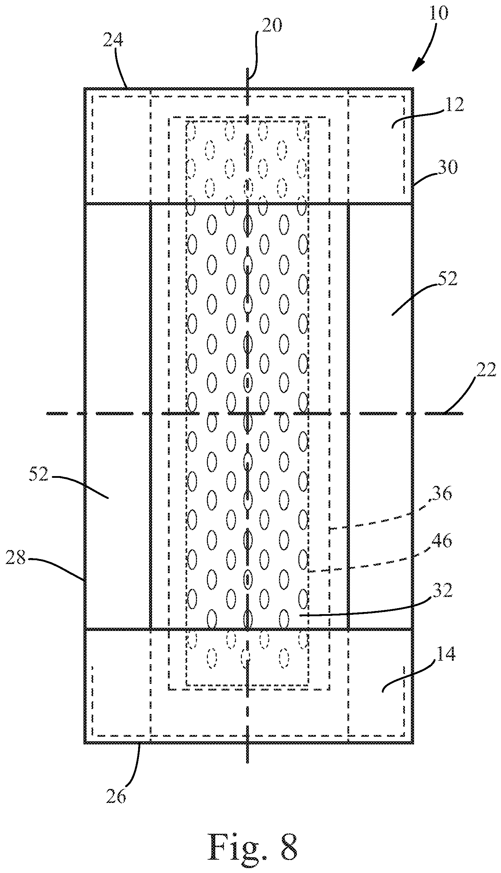

Referring to Fig. 8, which is not part of the claimed invention, a pair of raised barriers 52 may be attached to the topsheet 32, positioned under the topsheet 32, or formed from a portion of the topsheet 32 and/or acquisition or distribution materials. The raised barriers 52 may comprise foams, liquid impermeable materials, nonwoven materials, films, and/or other suitable materials. The raised barriers 52 may be "raised" relative to the topsheet 32 and may act in a similar fashion as the cuffs 16 and 17, thereby causing bodily exudates to remain over the absorbent core 36 such that they can be absorbed by the absorbent core 36. The raised barriers 52 may have any suitable thickness measured in a direction parallel to the central lateral axis 22, such as 2mm to 10mm, for example. In some instances, one raised barrier may be provided and this single raised barrier may fully surround, or partially surround, the absorbent core 36 or portions thereof. The raised barriers 52 may be raised a suitable amount from the topsheet 32 to contain the bodily exudates within the absorbent pad 10, such as in the range of 1mm to 30mm, or 2mm to 20mm, for example. Further details regarding raised barriers, including example structures and shape can be found in US2014/0171898 to Greening II, et al. The raised barriers 52 may be used with at least one pair of cuffs as well for better containment. The cuffs may be positioned laterally inboard or laterally outboard of the raised barriers. The raised barriers 52 or the cuffs 16, 17 may be referred to as "bodily exudate containment means".

As discussed above, one or more wetness guards 12, 14 may be provided on the absorbent pad 10. In general, the wetness guards 12 and 14 are provided to establish a barrier between the back, waist, and/or legs of the baby and portions of the topsheet 36 that are soiled with bodily exudates. A first wetness guard 12 is positioned on the first side of the central lateral axis 20 and a second wetness guard 14 is positioned on the second side of the central lateral axis 20. In some forms, other wetness guards may also be provided either in addition to the wetness guards 12 and 14, or in lieu of them. As an example, some wetness guards may extend longitudinally at least partially between the first end edge 24 and the second end edge 26. The wetness guards 12 and 14 may have any suitable width (measured in a direction parallel to the central lateral axis 22) and the length (measured in a direction parallel to the central longitudinal axis 20). In some instances, the wetness guards 12 and 14 may have a first width and the absorbent pad 10 may have a second width. The first width may be the same as or different than (larger or smaller) than the second width. For example, a wetness guard may only extend intermediate the cuffs 16 and not extend all the way to the first and second side edges 28 and 30. In some instances, one of the wetness guards 12 or 14 may have a first width and the other of the wetness guards 12 or 14 may have a second width. The first and second widths may be the same or different. In some instances, one of the wetness guards 12 or 14 may have a first length (measured in a direction parallel to the central longitudinal axis 20) and the other of the wetness guards 12 or 14 may have a second length. The first and second lengths may be the same or different. As illustrated in the figures, the first wetness guard 12 may have a longer length than the second wetness guard 14, or *vice versa.* In some instances, the wetness guards 12 and 14 may have the same length such that the absorbent pad 10 may be reversible.

The first wetness guard 12 may have a first end 54 and a second end 56. The first end 54 may be positioned proximate to the first end edge 24 of the absorbent pad 10. The second end 56 may be positioned intermediate the first end edge 24 and the central lateral axis 22. Although the second end 56 of the first wetness guard 12 is illustrated as being straight, it may also be concave or convex relative to the central lateral axis 22 or may have any other suitable shape. The first wetness guard 12 may be positioned over at least a portion of the topsheet 32 and may overlap or cross the central longitudinal axis 20. In other instances, the wetness guard 12 may be positioned over a portion of the topsheet 32 and portions of the cuffs 16, 17 or the raised barriers 52. The first wetness guard 12 may overlap a portion of absorbent core 36. The first wetness guard 12 may form a portion of a wearer-facing surface of the absorbent pad 10.

A first portion 58 of the first wetness guard 12 may be joined to the cuffs 16, 17, the raised barriers 52, the topsheet 32, and/or the backsheet 34 proximate to the first end edge 24 of the absorbent pad 10. A second portion 60 of the first wetness guard 12 may be joined to the cuffs 16, 17, the raised barriers 52, the topsheet 32, and/or the backsheet 34 proximate to the first side edge 28 of the absorbent pad 10. A third portion 62 of the first wetness guard 12 may be joined to the cuffs 16, 17, the raised barriers 52, the topsheet 32, and/or the backsheet 34 proximate to the second side edge 30 of the absorbent pad 10. The joining may comprise using mechanical joining or adhesive joining, for example. The joining may be intermittent or continuous. Through this joining, referring to Fig. 6, the second end 56 may be free of attachment to the topsheet 32, the cuffs 16, 17, and the raised barrier 52 such that a pocket is created at least between a non-wearer-facing surface of the first wetness guard 12 and the topsheet 32. In other instances, the second end 56 may be joined to, or partially joined to, the topsheet 32, the cuffs 16, 17, and/or the raised barriers 52, for example.

The second wetness guard 14 may have a first end 64 and a second end 66. The first end 64 may be positioned proximate to the second end edge 26 of the absorbent pad 10. The second end 66 may be positioned intermediate the second end edge 26 and the central lateral axis 22. Although the second end 66 of the second wetness guard 14 is illustrated as being straight, it may also be concave or convex relative to the central lateral axis 22 or may have any other suitable shape. The second wetness guard 14 may be positioned over at least a portion of the topsheet 32 and may overlap or cross the central longitudinal axis 20. In other instances, the wetness guard 14 may be positioned over the portion of the topsheet 32 and portions of the cuffs 16, 17 or the raised barriers 52. The second wetness guard 14 may overlap a portion of the absorbent core 36. The second wetness guard 14 may form a portion of a wearer-facing surface of the absorbent pad 10.

A first portion 68 of the second wetness guard 14 may be joined to the cuffs 16, 17, the raised barriers 52, the topsheet 32, and/or the backsheet 34 proximate to the second end edge 26 of the absorbent pad 10. A second portion 70 of the second wetness guard 14 may be joined to the cuffs 16, 17, the raised barriers 52, the topsheet 32, and/or the backsheet 34 proximate to the first side edge 28 of the absorbent pad 10. A third portion 72 of the second wetness guard 14 may be joined to the cuffs 16, 17, the raised barriers 52, the topsheet 32, and/or the backsheet proximate to the second side edge 30 of the absorbent pad 10. The joining may comprise using mechanical joining or adhesive joining, for example. The joining may be intermittent or continuous. Through this joining, the second end 66 may be free of attachment to the topsheet 32, the cuffs 16, 17, and the raised barrier 52 such that a pocket is created at least between a non-wearer-facing surface of the second wetness guard 14 and the topsheet 32. In other instances, the second end 66 may be joined to, or partially joined to, the topsheet 32, the cuffs 16, 17, and/or the raised barriers 52, for example.

In other instances, the first and/or the second wetness guards 12 and 14 may be joined to the topsheet 32, the cuffs 16, 17, the raised barriers 52, and/or the backsheet at any suitable locations on a wearer-facing surface of the absorbent pad 10. In an instance, all, or some of the perimeters of the wetness guards 12 and 14 may be joined to the wearer-facing surface. In other instances, all or some of non-perimeter areas may be joined to the wearer-facing surface. The two wetness guards 12 and 14, if both are provided, may be joined to a portion of the wearer-facing surface in the same fashion or in different fashions. In an instance, one or both of the wetness guards may be discrete components that are not joined to the absorbent pad in the package but instead are provided as a discrete component. Nurses or caregivers may then position the wetness guard or guards on portions of the wearer-facing surface of the absorbent pads 10 as appropriate. The wetness guards 12 and 14 may have a space between themselves and the first and second end edges 24 and 26. Stated another way, the most longitudinally outward portions of each wetness guard does not have to be positioned on the first end edges 24 or the second end edge 26, but instead a gap may exist, such as a 0.5 inch gap, for example.

The wetness guards 12 and 14 may have the same construction or a different construction. Referring to Fig. 7, the wetness guard 12 may comprise a laminate comprising a first liquid impermeable material (or substantially liquid impermeable material) 71 and a second liquid permeable material 73. The wetness guards may also comprise other liquid permeable or impermeable layers. The layers 71 and 73 may be joined together in any suitable fashion, such as through the use of an adhesive 75. The first liquid impermeable material 71 may comprise one or more nonwoven materials or films or a nonwoven material and a film as a laminate. The second liquid permeable material 73 may comprise one or more nonwoven materials or non-film materials. The first liquid impermeable material 71 may face the topsheet 32, while the second liquid permeable material 73 may face the wearer. As such, the first liquid impermeable material 71 may be used to create a barrier between the portion of the topsheet 32 under the wetness guards and the second liquid permeable material 73 to keep material 73 dry and sterile. The second liquid permeable material 73 may be used to provide a back, chest, or legs of a baby with a soft, dry, sterile, and comforting surface on which to be positioned. In an instance, the wetness guards 12 and 14 may comprise only one or more hydrophobic nonwoven materials without a liquid impermeable film. The hydrophobic nonwoven material(s) may be untreated, such that it remains naturally hydrophobic. In other instances, the hydrophobic nonwoven material(s) may be treated to become more hydrophobic.

The first and second wetness guards 12 and 14 may form a crotch receiving area 74 in the absorbent pad 10. The crotch receiving area 74 is the area between the two wetness guards 12 and 14 where a baby's waist and crotch area should be positioned when the baby is situated on the absorbent pad 10. The crotch receiving area 74 between the first and second wetness guards 12 and 14 may be any suitable size (length or width) for a particular size baby. A ratio of the surface area of the crotch receiving area 74 to the wetness guards 12 and 14 (together or separate if only one is provided) may be about 7:1, about 6:1, about 5:1, about 4:1, about 3:1, about 2:1, about 1:1, or about 0.5:1, or may be in the range of about 10:1 to about 1:1, specifically reciting all 0.1 increments of the ratios specified above and all ratios formed within the specified range. The crotch receiving area 74 may have an area in the range of about 50 cm² to about 500 cm², about 75 cm² to about 400 cm², about 100 cm² to about 300 cm², about 100 cm² to about 200 cm², about 100 cm² to about 175 cm², about 100 cm² to about 150 cm², or about 125 cm² to about 150 cm², for example, specifically reciting all 0.1 cm² increments within the specified ranges and all ranges formed therein or thereby.

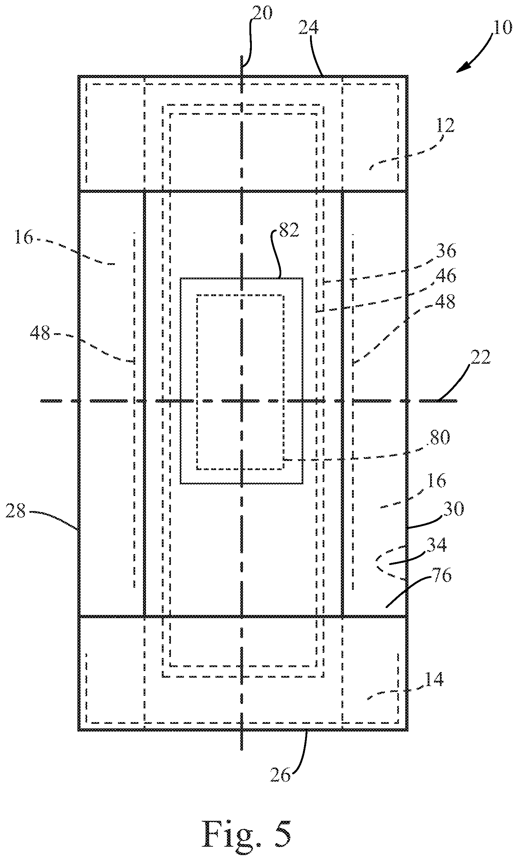

Referring to Figs. 5 and 7, an outer cover nonwoven material 76 may be positioned on a non-absorbent core-facing side of the backsheet 34. The outer cover nonwoven material 76 may be joined to the backsheet using an adhesive 84, for example. In such an instance, the outer cover nonwoven material 76 may contact the surface on which the absorbent pad 10 is to be placed. In instances where the outer cover nonwoven material 76 is not provided, the backsheet 34 may contact the surface on which the absorbent pad 10 is to be placed. The outer cover nonwoven material 76 may be formed of one or more layers and typically may be a soft material. The outer cover nonwoven material 76 may comprise a plurality of bonds, embossments, or three-dimensional features to provide a more consumer appealing appearance or for other reasons.

To create the first and/or second wetness guards 12 and 14, the backsheet 34 and outer cover nonwoven material 76 may extend beyond the first and second end edges 24 and 26 and be folded over the wearer-facing side of the absorbent pad 10. The folded over portions of the backsheet 34 and the outer cover nonwoven 76 may be joined to the topsheet 32, portions of the cuffs 16, 17, the raised barriers 52, and/or the backsheet 34 as described above. As such, the folded over portion of the backsheet 34 may form the first liquid impermeable material 71 and the outer cover nonwoven material 76 may form the second liquid permeable material 73 of the wetness guards 12 and 14.

In another instance, the first and/or second wetness guards 12 and 14 may be formed from discrete laminates each comprising the first liquid impermeable material 71 and the second liquid impermeable material 73. These discrete laminates may be joined to the topsheet 32, portions of the cuffs 16, 17, portions of the raised barriers 52, and/or to the backsheet 34 as described above.

Referring to Fig. 5, a non-slip means 80 or non-slip portion is provided on the outer cover nonwoven 76 or any other outermost layer of the absorbent pad 10 that is configured to contact the surface on which the absorbent pad 10 will be positioned. In an instance that the backsheet 34 is the outermost layer of the absorbent pad 10, a non-slip means 80 may not be required because of the gripping properties of a film. The non-slip means 80 is designed to hold the absorbent pad 10 stationary on the surface on which it is placed even during the baby's movement. The non-slip means 80 may have any suitable size or shape. For instance, the non-slip 80 means may be a pattern of lines, a pattern of dots, a pattern of shapes, only one area, and/or more than one area, for example. The non-slip means 80 may be an adhesive covered by a release paper or a releasable material 82. The release paper or the releasable material 82 may be used to prevent the absorbent pads 10 from sticking to each other while in the package or sticking to surfaces until positioned on the desired surface. Other forms of non-slip means 80 may comprise high coefficient of friction areas, ridges, bumps, and/or wrinkles, for example.

The absorbent pad 10 may have a wearer-facing surface area (the entire wearer-facing surface including areas of the wetness guards) in the range of about 150 cm² to about 1,500 cm², about 175 cm² to about 1,000 cm², about 200 cm² to about 800 cm², about 200 cm² to about 500 cm², about 200 cm² to about 400 cm², about 200 cm² to about 300 cm², about 200 cm² to about 250 cm², about 210 cm² to about 240 cm², or about 225 cm², for example, specifically reciting all 0.1 cm² increments within the specified ranges and all ranges formed therein or thereby.

The wetness guards 12 and/or 14 may each have an area in the range of about 10 cm² to about 800 cm², about 15 cm² to about 600 cm², about 15 cm² to about 400 cm², about 20 cm² to about 300 cm², about 20 cm² to about 200 cm², about 20 cm² to about 100 cm², about 20 cm² to about 75 cm², or about 25 cm² to about 70 cm², for example, specifically reciting all 0.1 cm² increments within the specified ranges and all ranges formed therein or thereby. As mentioned above, the wetness guard 12 may have an area that is the same or different than the wetness guard 14, if both are provided in an absorbent pad.

The wearer-facing surface or the garment-facing surface of the absorbent pad may also comprise one or more wetness indicators and one or more lotions.

Dots in Fig. 7 also illustrate examples of various construction adhesives joining various layers or components.

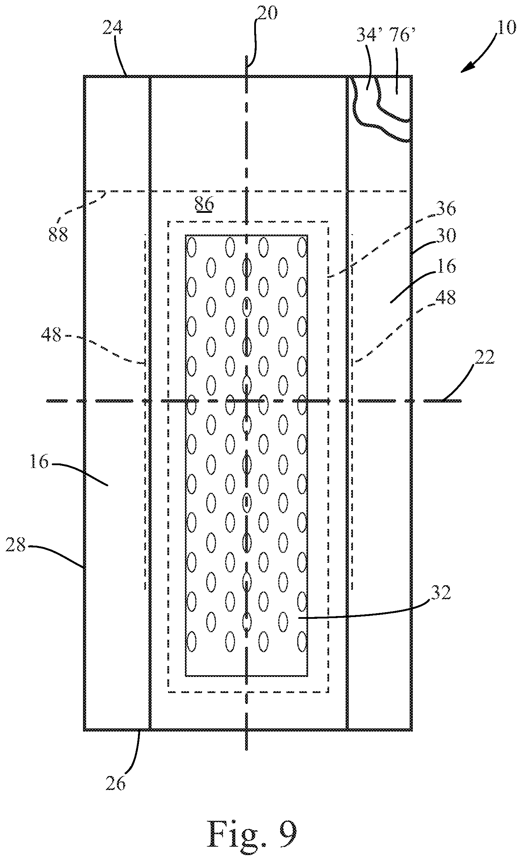

In some instances outside the claimed invention, referring to Fig. 9, the first and second wetness guards 12 and 14 may not be provided and instead, the absorbent pad 10 may be configured such that a nurse or caregiver may fold a portion of the backsheet 34' and a portion of the outer cover nonwoven material 76' about a fold line 88 to create a wetness guard, formed of the portion of the backsheet 34' and the portion of the outer cover nonwoven material 76'. The portion of the backsheet 34' and the portion of the outer cover nonwoven material 76' may be folded towards a wearer-facing surface 86 of the absorbent pad 10. In some instances, the fold line 88 may be embossed or may be a weakened area in the absorbent pad 10 to enable such folding. Although the fold line 88 is only illustrated on one side of the lateral axis 22 in Fig. 9, a fold line could also be provided on the other side of the lateral axis 22 or on both sides of the lateral axis 22. In an example, a wetness guard (like in Fig. 3) may be provided on one side of the lateral axis 22 and the folded over configuration of a wetness guard may be provided on a second side of the lateral axis 22. In any event, when the folded over configuration of the wetness guard is provided, the absorbent core 36 may be shortened to allow for easier folding.

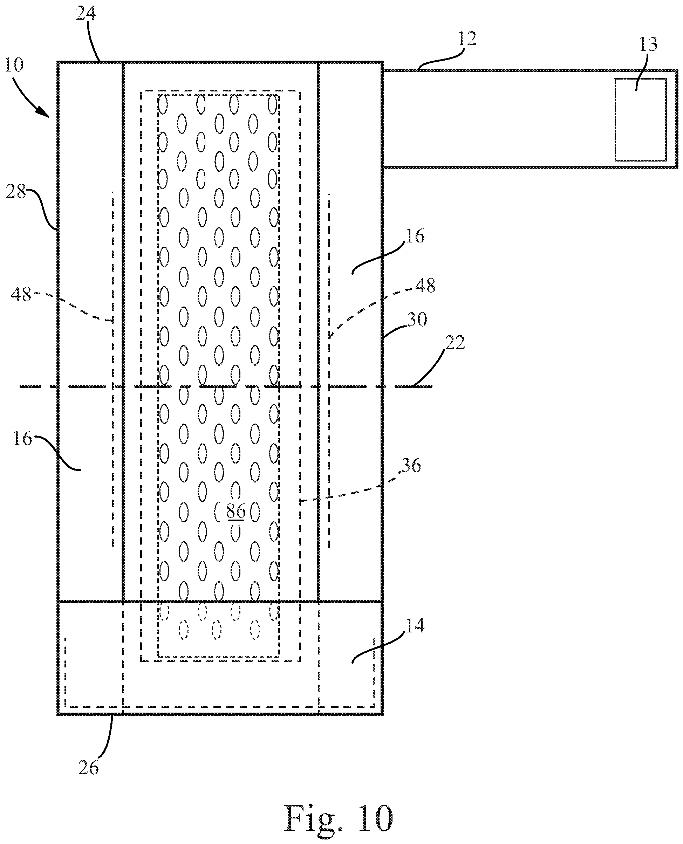

In another instance, referring to Fig. 10, at least one of or both of the wetness guards 12 and 14 may only be attached to the first side edge 28, the second side edge 30, and/or a portion of the absorbent pad 10 proximate to one of the side edges 28 and 30, for example. In other instances, the wetness guards 12 and/or 14 may be attached to the outer cover nonwoven material 76, the backsheet 34, the topsheet 32, one of the raised barriers 52, and/or one of the cuffs (16 or 17), for example. In such an instance, the wetness guard 12 in Fig. 10 may be configured to be folded over into the position illustrated in, for example Fig. 3. The wetness guard 14 may have a similar configuration as the illustrated wetness guard 12 or may be provided as described with reference to Fig. 3. The wetness guard 12 (and/or the wetness guard 14 if provided in the same configuration) may comprise a fastener 13, such as a plurality of hooks or an adhesive, for example, such that when the wetness guard 12 is folded over the absorbent pad 10, it can be joined by the fastener 13 to a portion of the wearer-facing surface 86 of the absorbent pad 10. The fastener 13 may help maintain the wetness guard 12 in position during use of the absorbent pad 10. The materials of the wetness guard 12 may be the same as or similar to that described above.

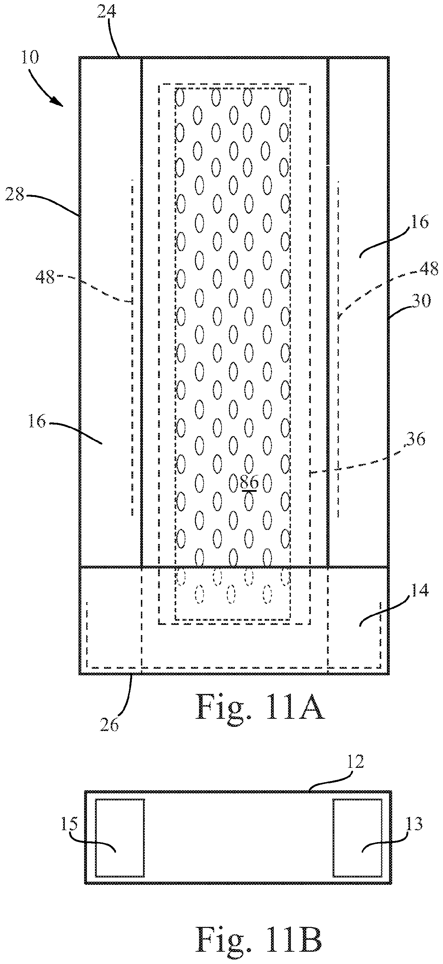

In still other instances, referring to Figs. 11A and 11B, one or both of the wetness guards 12 and 14 may be a discrete component (Fig. 11B) from the absorbent pad 10 (Fig. 11A) and may be configured to be placed on the absorbent pad 10, in for example, the positions illustrated in Fig. 3, or in other positions as desired by the nurse or caregiver. In some instances, as illustrated in Figs. 11A and 11B, one wetness guard 12 may be a discrete component and the other wetness guard 14 may be joined to the cuffs 16, the raised barrier 52, the topsheet 32, and/or the backsheet 34. The discrete wetness guard or guards may be packaged with the absorbent pads or may be packaged and/or sold separately. The discrete wetness guard 12 (and/or the wetness guard 14, if discrete) may have a first fastener 13 and a second fastener 15. The first and second fasteners 13 and 15 may be used to join the discrete wetness guard 12 to a portion of the wearer-facing surface 86 as desired and maintain the wetness guard 12 in position during use of the absorbent pad. The materials of the wetness guard may be the same as or similar to that described above.

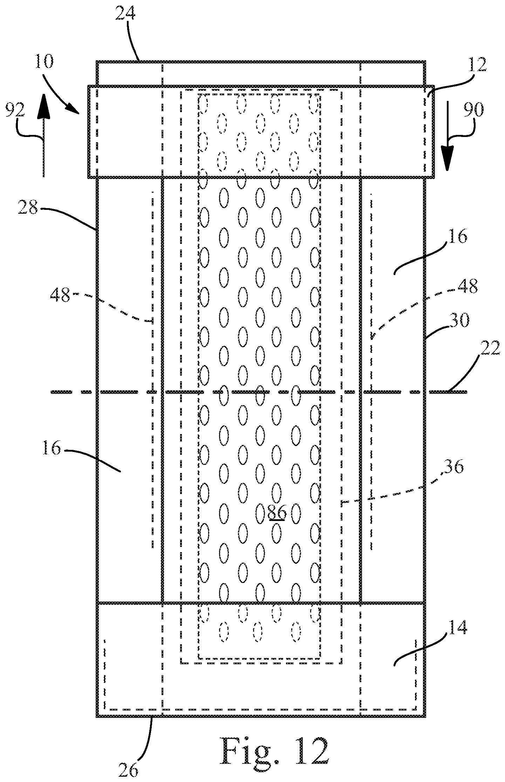

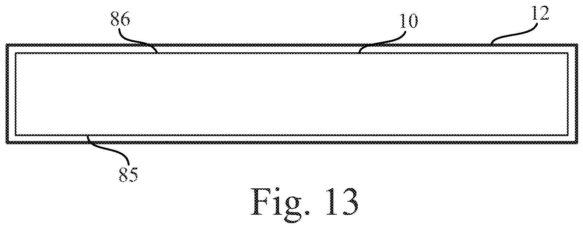

In yet other instances, referring to Figs. 12 and 13, at least one of, or both of, the wetness guards 12 and 14 may be provided in a sleeve form. The sleeve may be positioned on the absorbent pad 10 in a package, positioned separately from the absorbent pad 10 in the package, or may be sold in a separate package. Once positioned on the absorbent pad, the sleeve may be moveable in the directions of arrows 90 and 92 to allow a nurse or caregiver to position the wetness guard 12 as desired. In some instances, as illustrated in Fig. 12, one wetness guard 14 may be joined to the wearer-facing surface 86 and another wetness guard 12 may take the form of a sleeve. In some forms, the materials of the wetness guard may be the same as or similar to that described above. In other forms, only portions of the wetness guard positioned over the wearer-facing surface 86 may have the wetness guard constructions described herein, with other portions (i.e., portions wrapped around a non-wearer-facing surface 85 of the absorbent pad 10) being constructed of other materials, such as nonwoven materials without films, for example. Fig. 13 illustrates an example of how the wetness guard 12 in the form of a sleeve would surround the absorbent pad 10.

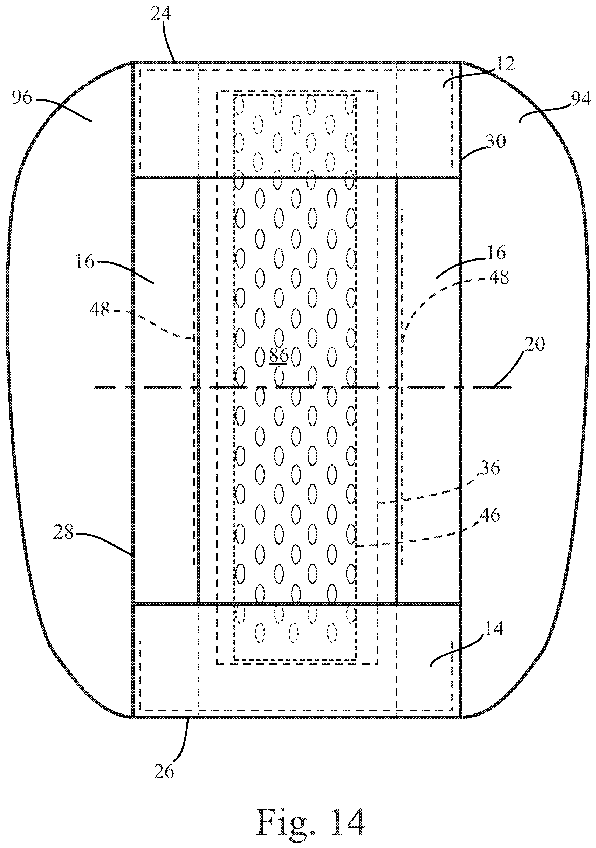

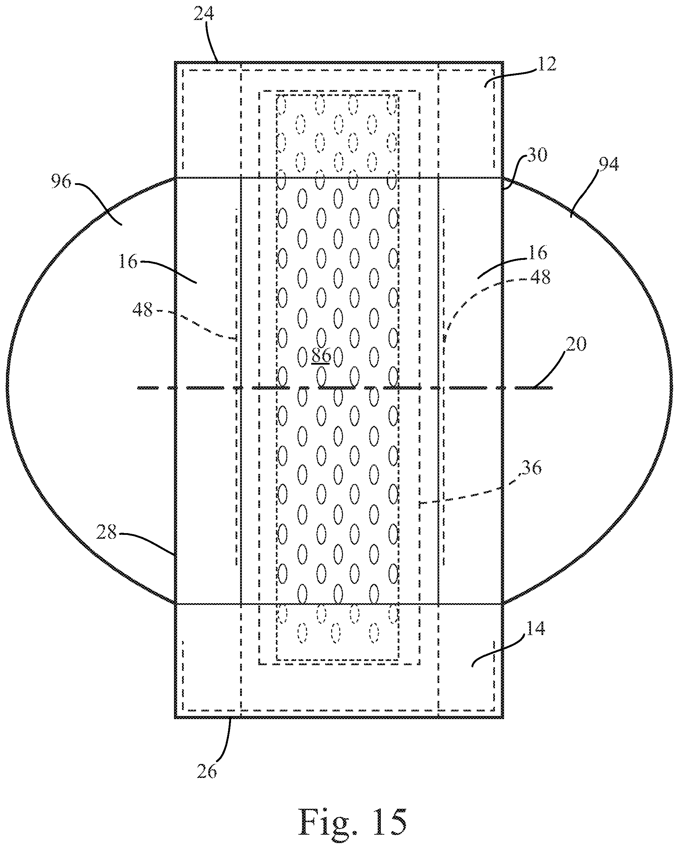

Any of the absorbent pads 10 of the present disclosure may have leakage guards 94 and 96 that extend outwardly from the first and second side edges 28 and 30, respectively. The leakage guards 94 and 96 may extend laterally outwardly from all of (Fig. 14) or portions of (Fig. 15) the first and second side edges 28 and 30, respectively. The leakage guards 94 and 96 may have any suitable shape. The leakage guards 94 and 96 may be configured to prevent, or at least inhibit, soiling of areas adjacent to the absorbent pad 10 in instances where bodily exudates may leak from the side edges 28 and 30 of the absorbent pad 10. If the absorbent pad 10 is being used for babies that are on feeding tubes, IVs, or other liquid supplements (hereafter "supplements"), tubes or needles that provide these supplements may leak in areas adjacent to the absorbent pad 10. The leakage guards 94 and 96 may be provided to contain this leakage and prevent, or at least inhibit, soiling of surfaces adjacent to the absorbent pads 10. In some instances, the leakage guards 94 and 96 may be used to essentially funnel (if raised relative to the absorbent pad 10) bodily exudates or supplements back towards the absorbent pad 10, such that they may be absorbed by the absorbent pads 10.

The leakage guards 94 and 96 may comprise one or more films. In other instances, the leakage guards 94 and 96 may comprise one or more films and one or more nonwoven materials. In still other instances, the leakage guards 94 and 96 may comprise an extension of the backsheet 34, an extension of the topsheet 32, (an optional acquisition material or extension of acquisition material 46) and an extension of the absorbent core 36, for example, to make the leakage guards absorbent. In other instances, the leakage guards 94 and 96 may comprise a film, an absorbent core or absorbent material, an optional acquisition material, and a nonwoven material, with the absorbent core or absorbent material (and optional acquisition material) being positioned between the film and the nonwoven material. If an absorbent core or absorbent material is provided in the leakage guards 94 and 96, the leakage guards may be used to not only contain bodily exudates and supplements, but also to absorb them. Leakage guards may also be designed to extend outwardly from the first and second end edges 24 and 26. In other forms, a single leakage guard may fully, or at least partially, surround the absorbent pad 10. In other instances, the leakage guard may be made of a separate piece of material or materials, onto which the absorbent pad is placed. Any of the configurations of the leakage guards herein may be liquid impermeable or substantially liquid impermeable.

The absorbent pads of the present disclosure may be sold or displayed in arrays or on-line arrays. The arrays or on-line arrays may comprise different sizes of the absorbent pads or absorbent pads with different features. In other instances, the arrays may comprise absorbent pads (same or different sizes) and diapers (same or different sizes). The absorbent pads of the present disclosure may be packaged with the diapers or packaged separately from the diapers. The absorbent pads may not be bi-folded or tri-folded in a package, but instead may be packaged flat.

Any configurations of the wetness guards 12 and 14 described herein may be used together, for example, a wetness guard that is discrete may be used with a wetness guard that folds over, or a wetness guard that is attached at only one side of the absorbent pad 10 may be used with a permanent or discrete wetness guard. Further, any configurations of the wetness guards described herein may be used with any of the configurations of the leakage guards described herein. Other features of the absorbent pads may be used in combination with any of the wetness guard configurations described herein or any of the leakage guard configurations described herein. Even the sleeve configuration of a wetness guard described herein may be used with an absorbent pad 10 having leakage guards as long as the sleeve-type wetness guard was wide enough to slide over the leakage guards.

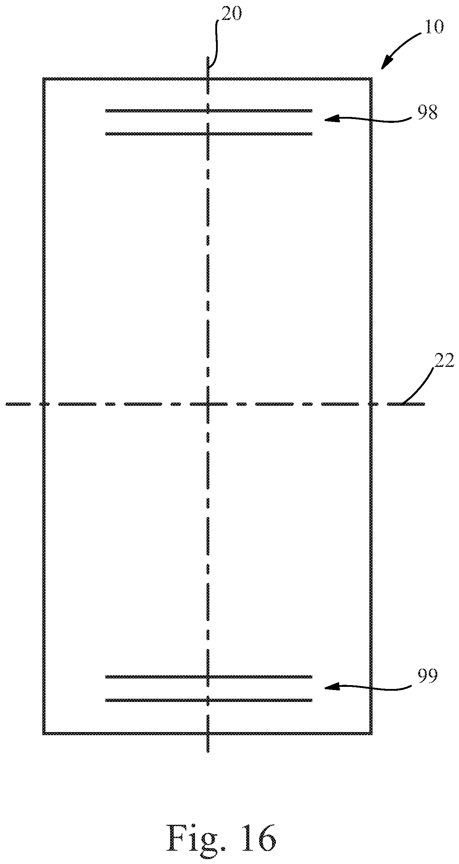

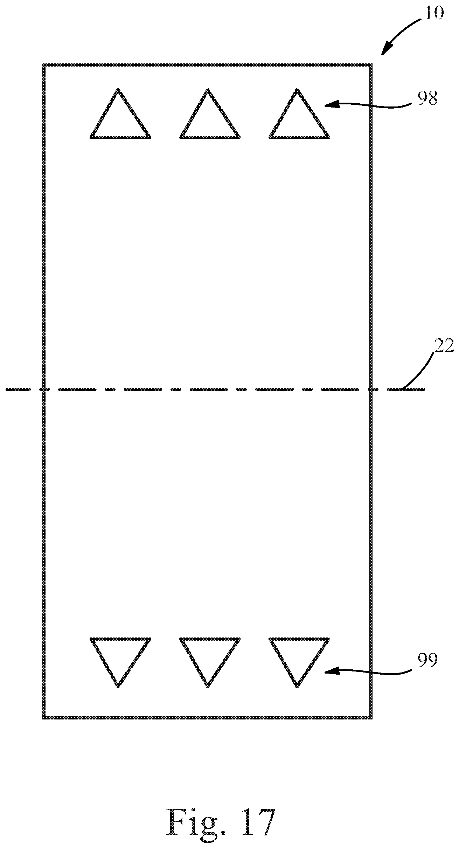

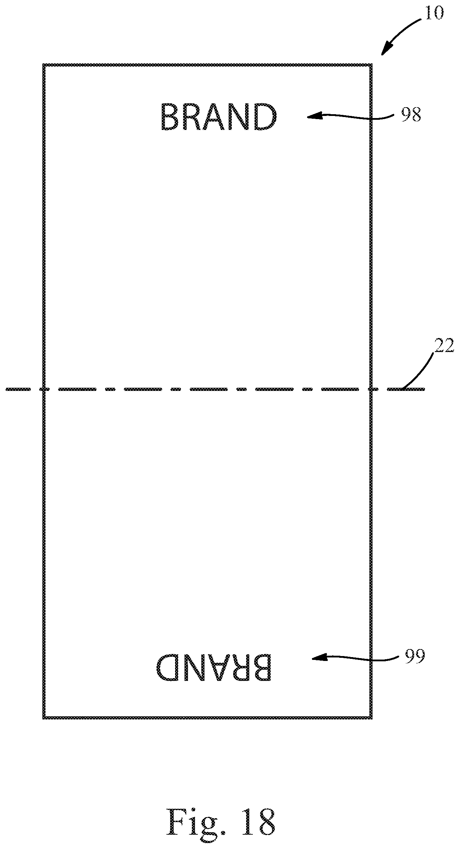

Figs. 16-18 are schematic illustrations of absorbent pads 10 with graphics and/or indicia 98, 99 (hereafter "graphics"), garment-facing surface facing the viewer. The graphics 98 may be on a first side of the central lateral axis 22 and viewable from the garment-facing surface and the graphics 99 may be on a second side of the central lateral axis 22 and viewable from the garment-facing surface. The graphics 98 and/or 99 may or may not cross the central longitudinal axis. The graphics 98 and/or 99 may be a single graphic or multiple graphics that form a single image, multiple images, single patterns or multiple patterns. The graphics 98 may be a mirror image of the graphics 99, relative to the central lateral axis 22 to indicate that the absorbent pad 10 is reversible to the caregiver. In this instance, the graphics provide functional features. In other instances, the graphics 98 may not be a mirror image of the graphics 99, relative to the central lateral axis 22. Referring to Fig. 18, the graphics 98 and/or 99 may be at least partially comprised of brand names, characters, and/or logos. The graphics 98 and/or 99 may be printed on, positioned on, and/or applied to the backsheet 34 and/or the outer cover nonwoven material 76, for example. If the graphics 98 and 99 are on the backsheet 34, the outer cover nonwoven material 76 may have an opacity such that the graphics 98 and 99 are viewable therethrough. The graphics 98 and 99 may be on a garment-facing side of the backsheet 34 and/or on the wearer-facing side of the backsheet 34. By having graphics 98 and 99 that are mirror images of each other, relative to the central lateral axis 22, the absorbent pad 10 may be reversible. Stated another way, a baby's (or wearer's) back may rest on graphics 98 or 99 depending on how the absorbent pad 10 is placed on a horizontal surface and how the baby is positioned on the absorbent pad 10. If the absorbent pad 10 is meant to be reversible, the absorbent material within the absorbent core 36, in some instances, may be uniform and homogeneous and not have an absorbent material gradient.

In some instances, it may be desirable for the absorbent pads to be free of fragrances, perfumes, and/or lotions. In other instances, one or more components of the absorbent pads, such as the topsheet, for example, may be free of fragrances, perfumes, and/or lotions, The absorbent pads 10 of the present disclosure may comprise or may be used with one or more fastening members used to fasten one portion of the absorbent pad 10 to another portion of the absorbent pad 10. In certain instances, it may be desirable for a caregiver or nurse to at least partially fold or at least partially wrap the absorbent pad 10 around at least a portion of a torso area of a wearer. In such an instance, the caregiver may want to fasten a first portion of the absorbent pad 10 to a second portion of the absorbent pad 10 for purposes of containing bodily exudates. In some cases, the caregiver or nurse may want to use two absorbent pads 10 and at least partially fasten them together. In such an instance, one absorbent pad may be laid on a horizontal surface, a baby's back side crotch region may rest of the absorbent pad, and another absorbent pad may be placed on a front side of the baby's crotch region. One or more fastening members may be used to at least partially attach the two absorbent pads together. In certain circumstances, referring to Fig. 4, the absorbent pad 10 may be provided with one or more fastening members 4 comprising one or more fasteners 6. The fastening members 4 may be permanently joined to the absorbent pad 10, like a taped diaper, for example. The fastening members 4 may be provided at any suitable location proximate to a perimeter of the absorbent pad 10.

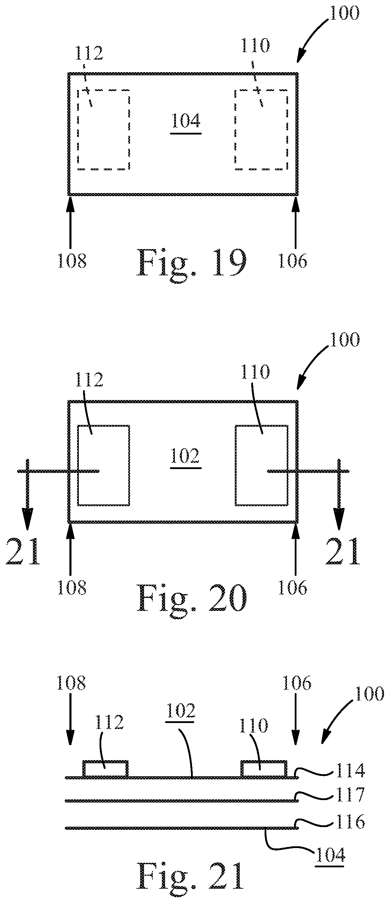

In other instances, the fastening members may be discrete fastening members that are provided or sold either with or separately from the absorbent pads 10. Any number of these fastening members may be used with one or more absorbent pads 10. Referring to Figs. 19-21, various example discrete fastening members 100 that may be used with one or more absorbent pads 10 of the present disclosure are illustrated. The fastening members 100 may or may not be or comprise stretch panels or elastic members. The fastening members 100 may each comprise a first surface 102, a second surface 104, a first end 106, and a second end 108. The first surface 102 may be opposite to the second surface 104 and the first end 106 may be opposite to the second end 108. The fastening members 100 may comprise a first fastener 110 configured to engage a first portion of the outer cover nonwoven material 76 or one of the wetness guards 12, 14 and positioned on the first surface 102. The fastening members 100 may comprise a second fastener 112 configured to engage a second, different portion of the outer cover nonwoven material 76 or one of the wetness guards 12 and positioned on the first surface 102. In some instances, it may be desirable to provide the first fastening member 110 on the first surface 102 and the second fastening member 112 on the second surface 104, so that the fastener can be folded into a loop to hold a tube, for example. The fastener 110 may be positioned proximate to the first end 106 and the fastener 112 may be positioned proximate to the second end 108. In some instances, one or more fully removable fastening members 100 may be provided with an absorbent pad 10 and another fastening member may be permanently joined to the absorbent pad, for example.

Referring to Figs. 19-23, the first fastener 110 and the second fastener 112 may not extend to the perimeter of the fastening members 100. This may help prevent, or at least inhibit, rough fastener material (e.g., hooks) from contacting or irritating a wearer's skin. The fasteners 110 and 112 are illustrated as rectangular but may be any other suitable shape, such as circular or ovate, for example. In some instances, it may be desirable to have fasteners without corners to again prevent, or at least inhibit the fasteners from at irritating a wearer's skin. In a package, the first and second fasteners 110 and 112 may be joined to the first surface 102 so they are at least inhibited from unintentionally engaging other portions of the absorbent pads, other fastening members, and/or other items.

Referring to Fig. 21, the fastening members 100 may comprise a first nonwoven or other substrate 114, a second or other nonwoven substrate 116, and an elastic material 117 positioned at least partially intermediate the first and second substrates 114 and 116. The elastic material may comprise an elastic nonwoven material, an elastic film, and/or elastic strands, for example. In other instances, the fastening members may comprise one or more substrates and may not comprise an elastic material.

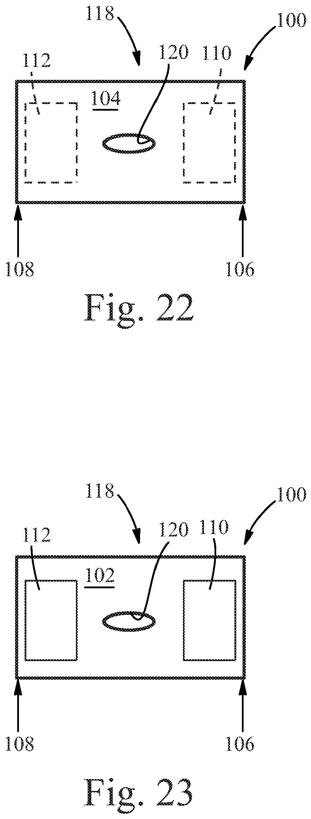

Referring to Figs. 22 and 23, a middle portion 118 (e.g., between the first and second ends 106 and 108) of the fastening member 100 may define one or more slots 120 or apertures therein. The slots or apertures 120 may have any suitable size and/or shape. In certain instances, premature, NAS babies, and other babies are on feeding tubes, corded monitoring device (e.g., heart rate monitor), corded life support device, or the like. These tubes and cords may be positioned through the slots 120 or apertures to help hold the tubes and cords in place. In some instances, the middle portion 118, or other portions, of the fastening member 100 may be formed of a highly breathable material, such as a highly breathable film, nonwoven, film/nonwoven laminate, or an apertured film or apertured nonwoven material, for example.

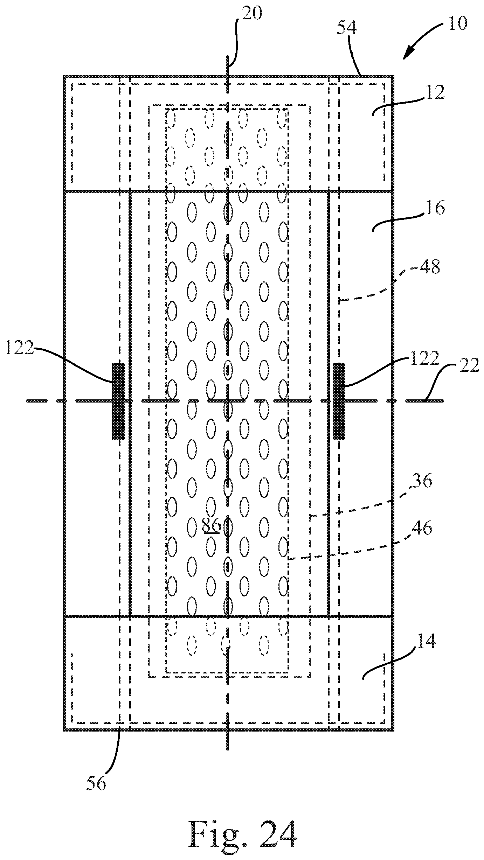

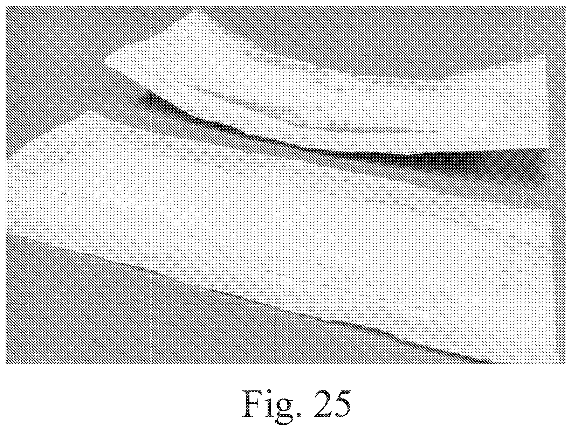

Referring to Fig. 24, the elastics 48 are only be joined to the cuffs 16 in a joined area 122 which is a longitudinally central joined area relative to the central lateral axis 22 of the absorbent pad 10. The elastics 48 may not extend the full longitudinal direction of the absorbent pad 10 after the absorbent pad is separated from other absorbent pads at the first end 54 and at the second end 56. In this instance, the elastics 48 may "snap back" toward the joined area 122. Portions of the elastics 48 outside of the joined area 122 may be free of joinder or attachment to the cuffs 16, thereby not applying elastic forces to areas of the cuffs 16 outside of the joined area 122. The joined area 122 may be centrally located on the elastics 48 in the longitudinal direction (i.e., a direction about the longitudinal axis 20). Stated another way, the joined area 122 may exhibit symmetry with respect to the lateral axis 22. In other cases, the joined area 122 may not exhibit symmetry with respect to the lateral axis 22 or may not even cross the lateral axis 22. In this instance, the joined area 122 may be fully positioned on a first side of the lateral axis 22 or may be fully positioned on a second side of the lateral axis 22. More than one joined area 122 may exist for a single cuff 16. In a single elastic 48, one joined area may be on the first side of the lateral axis 22 and a second joined area may be on a second side of the lateral axis 22. The joined areas 122 may have a length extending in a direction generally parallel to the longitudinal axis 20 of about 0.25 inches to about 10 inches, about 0.5 inches to about 5 inches, about 0.5 inches to about 4 inches, about 0.5 inches to about 3 inches, about 0.5 inches to about 2 inches, less than about 2 inches, less than about 1.75 inches, less than about 1.5 inches, about 0.5 inches to about 1.75 inches, about 0.5 inches to about 1.5 inches, about 0.5 inches to about 1.25 inches, or about 0.5 inches to about 1 inch, specifically reciting all 0.1 inch increments with the specified ranges and all ranges formed therein or thereby. The certain longitudinal length of the joined areas 122 in a particular absorbent pad 10 may depend on the size of the absorbent pad 10 and/or the intended use of the absorbent pad 10. The joined area 122 may comprise one or more adhesives and/or one or more mechanical, ultrasonic, and/or thermal bonds, for example, to join the elastics 48 in the joined area 122 to the cuffs 16. The joined areas 122 are illustrated as a rectangular block for illustration purposes only, and other suitable shapes are within the scope of the present disclosure. The elastics 48 may be under a pre-strain when joined to the cuffs 16 to activate the joined areas 122 when the pre-strain is released. In some instances, the elastics 48 may only extend longitudinally through the joined area 122 and may be pre-strained. The cuffs 16 may be formed of one or more materials or nonwoven materials. If only one material is used, that material may be folded over itself to enclose the elastics 48. If two materials are used, the materials may sandwich the elastics therebetween. The joined area 122 may be two or more discrete bonds with pre-strained elastics 48 therebetween. The elastics 49, if provided, may have the same or different features as the elastics 48 described in this paragraph. In other instances, only the elastics 49 may have the features described in this paragraph with respect to the elastics 48. By providing elastics 48 and/or 49 with one or more joined areas 122, the longitudinal end portions of the absorbent pad 10 may be slightly raised when the absorbent pad 10 is positioned on a flat, horizontal surface, wearer-facing side facing away from the flat, horizontal surface. The raising of the longitudinal end portions may provide better bodily exudate containment. An example absorbent pad having raised longitudinal end portions (top) compared to a flat absorbent pad (bottom) is illustrated in Fig. 25.

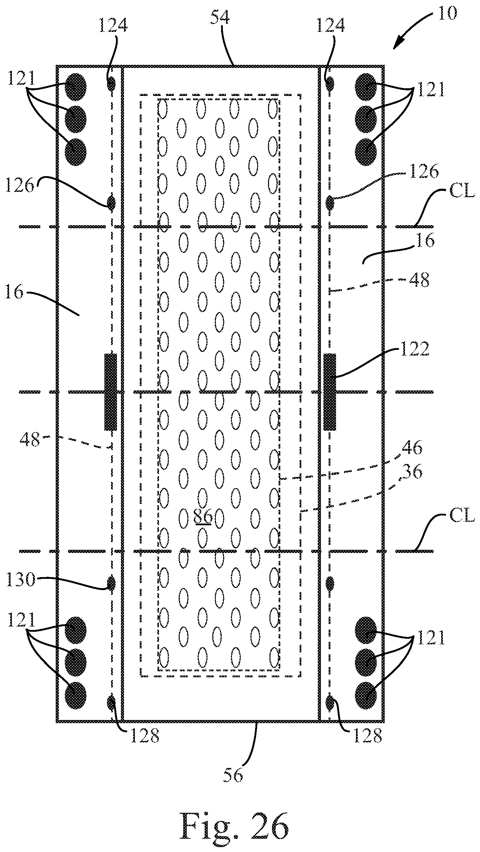

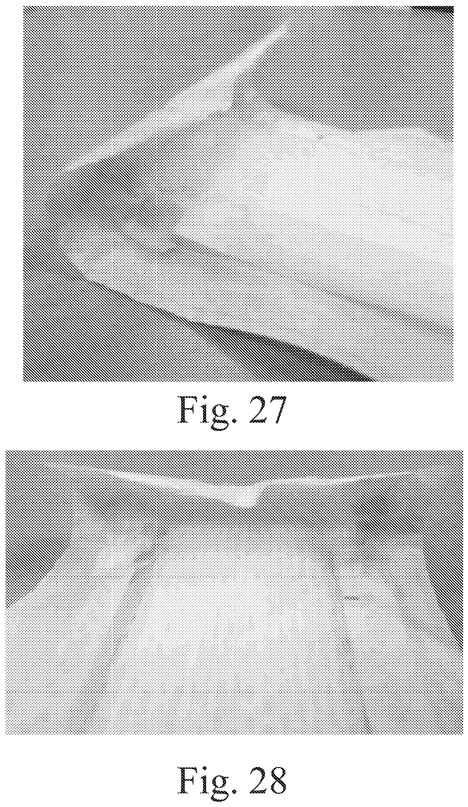

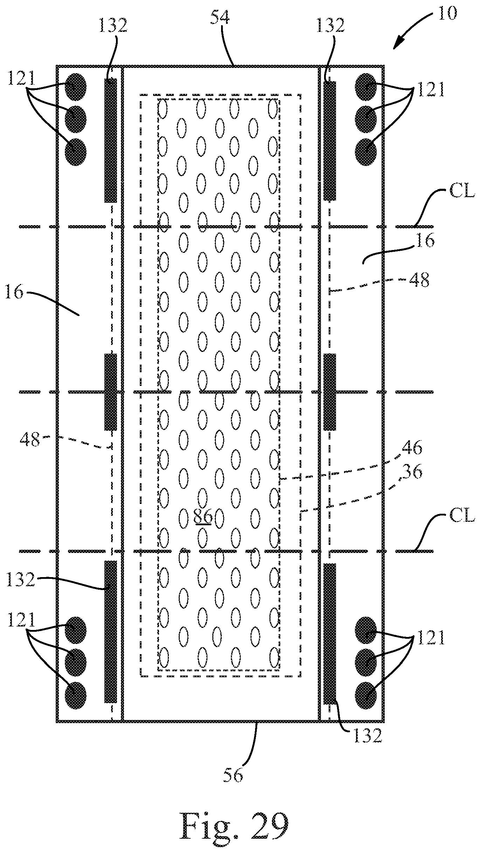

Referring to Fig. 26, an absorbent pad 10 may have cuffs 16 with a certain elastic configuration. The elastics 48 may or may not have the joined areas 122 discussed above. The elastics 48 may each have a first bond 124 proximate to the first end 54 and a second bond 126 positioned more distal from the first end 54 than the first bond 124. The elastics 48 may also each have a third bond 128 proximate to the second end 56 and a fourth bond 130 positioned more distal from the second end 56 than the third bond 128. The elastics 48 may be cut (example cut lines "CL" illustrated in dash in Fig. 26) intermediate the second bond 126 and joined area 122 or, if the joined area 122 is not provided, intermediate the second bond 126 and the fourth bond 130. If the joined area 122 is provided, the elastics 48 may also be cut intermediate the fourth bond 130 and the joined area 122. The bonds 124 and 126, owing to contraction of the elastic 48 therebetween, may cause the first end 54 to curl or fold over a portion of the wearer-facing surface 86 proximate to the first end 54. The bonds 128 and 130, owing to the contraction of the elastic 48 therebetween, may cause the second end 56 to curl or fold over a portion of the wearer-facing surface 86 proximate to the second end 56. These curled or folded over portions may help contain bodily exudates on the absorbent pad 10 so that they may be absorbed by the absorbent core 36 and so that surfaces adjacent to the absorbent pad 10 are not soiled. Fig. 27 is a perspective side view of a curled or folded over portion of an absorbent pad. Fig. 28 is another perspective view of the curled or folded over portion of the absorbent pad of Fig. 28. These features may be used with or without the various wetness guards 12, 14 disclosed herein. In other instances, these curled or folded over portions may be used to create the wetness guards 12, 14 when they are curled or folded over. When used as wetness guards, the curled or folded over portions may be tacked, bonded, and/or glued to the wearer-facing surface to hold them in place. The elastics 49 of the cuffs 17, if provided, may have the similar or the same features as the elastics 48 of the cuffs 16 described in this paragraph. The bonds 124, 126, 128, and 130 may comprise adhesive bonds, mechanical bonds, ultra-sonic bonds, and/or thermal bonds, for example. One or more tack down bonds 121 may also be present on the absorbent pad 10. The tack down bonds 121 may be used to join the cuffs 16 to the topsheet or to other portions of the absorbent pad 10. Referring to Fig. 29, instead of providing the first, second, third, and fourth bonds 124, 126, 128, and 130, a continuous bond 132 may be provided in at least one end region of the elastics 48. The continuous bond 132 may function like the first, second, third, and fourth bonds 124, 126, 128, and 130 described above and may result in curled or folded portions illustrated in Figs. 27 and 28. The continuous bonds 132 may comprise adhesive bonds, mechanical bonds, ultra-sonic bonds, and/or mechanical bonds, for example. Continuous bonds may also be provided on the elastics 49 of the cuffs 17, if provided. The elastics may be pre-strained before the continuous bond is applied such that elastic contraction within the continuous bonds may cause the end portions to curl or fold over.

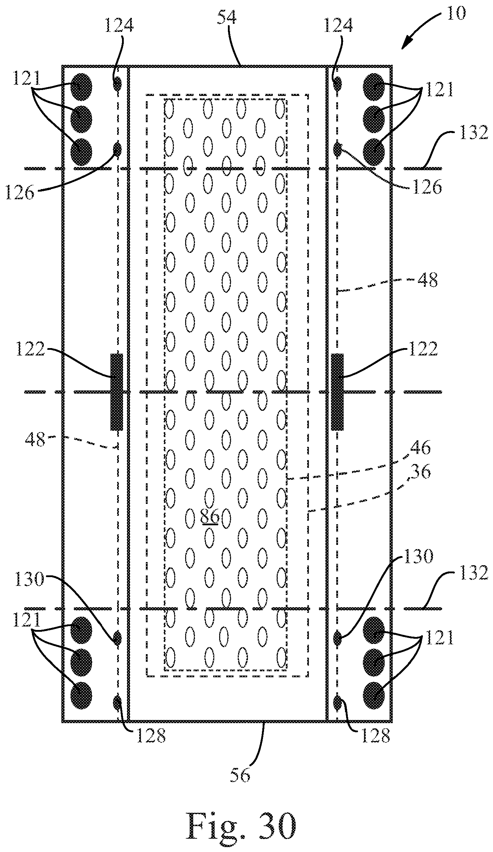

Referring to Fig. 30, portions of the absorbent core 36, the absorbent material 38 within the absorbent core 36, and/or other layers of the absorbent pads may comprise one or more fold lines 132 to allow easier folding of portions of the absorbent pad 10. The fold lines 132 may be areas with reduced or no absorbent material 38 or embossed areas, for example. The fold lines may be at any suitable locations within the absorbent core 36 and/or the absorbent pad 10. The fold lines may be helpful to a caregiver or nurse when situating the absorbent pad 10 at least partially around a baby, for example.

Methods for producing cuffs of the absorbent pads are also disclosed. The cuffs may be made online or by hand. In an online context, a first nonwoven web or a first web may be advanced (e.g., on a conveyor) in a machine direction. One or more elastics may be applied in a pre-strained condition to the first nonwoven web. The pre-strained elastic may have the same machine direction length as the first nonwoven web. The pre-strained elastic may be joined to the first nonwoven web in a plurality of joined areas 122 (e.g., one or more joined areas per discrete cuff length). In some instances, the pre-strained elastic may only be joined to the first nonwoven web in the joined areas 122. The joining may use adhesives or bonds, as discussed herein. The remainder of the elastic outside of the joined areas 122 may be free of joinder to the first nonwoven web. The first nonwoven web may then be folded over itself to enclose the elastic. In other instances, a second nonwoven web or second web may be positioned over the first nonwoven web and may be joined to the first nonwoven web to enclose the elastic. The second nonwoven web may also be joined to the elastic in the joined areas 122. The formed web of cuffs may then be cut to suitable discrete lengths. When the web of cuffs is cut to suitable discrete lengths, the elastic in each cuff may "snap back" towards the joined areas 122, leaving the elastic only pre-strained in the joined area, with the remainder of the elastic being non-pre-strained and not attached to the first and/or second nonwoven discrete webs. The cuffs may then be attached to an absorbent pad. Once the cuffs are attached to an absorbent pad, the structure of the top photo of Fig. 25 may be created. In some instances, the first nonwoven web may first be joined to a moving web comprising a top sheet, a backsheet, and an absorbent core positioned at least partially therebetween, among other components (e.g., an acquisition layer positioned intermediate the topsheet and the absorbent core). In this instance, the cuffs and the moving web may be cut into discrete absorbent pads at the same time.

In addition to being joined to the first and/or second nonwoven webs in the joined areas 122, the elastics may also be joined to the first and/or second nonwoven webs at bonds (e.g., bonds 124, 126, 128, and 130 or continuous bonds 132). These features may apply in a single web cuff that is folded over itself or to a two web cuff. Also, the elastics may only be joined to the first and/or second nonwoven webs at the bonds and not in the joined areas 122. In any event, if the elastics are joined to the bonds (e.g., 124 and 126), portions of the elastics intermediate the bonds will remain pre-strained after the elastics are cut intermediate bond pairs (e.g., "CL" of Fig. 26) and after the cuffs are cut into suitable discrete lengths. This may cause the pre-strained elastic portions intermediate the bonds (e.g., 124 and 126), to contract upon release of the pre-strain force and cause the curling or folding of longitudinal end portions as illustrated in Fig. 27.

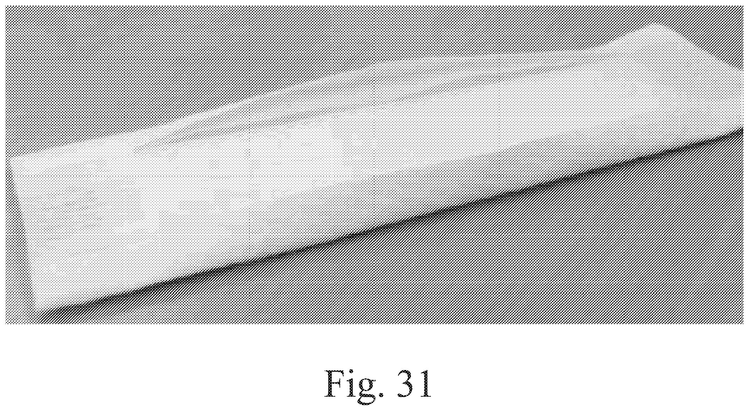

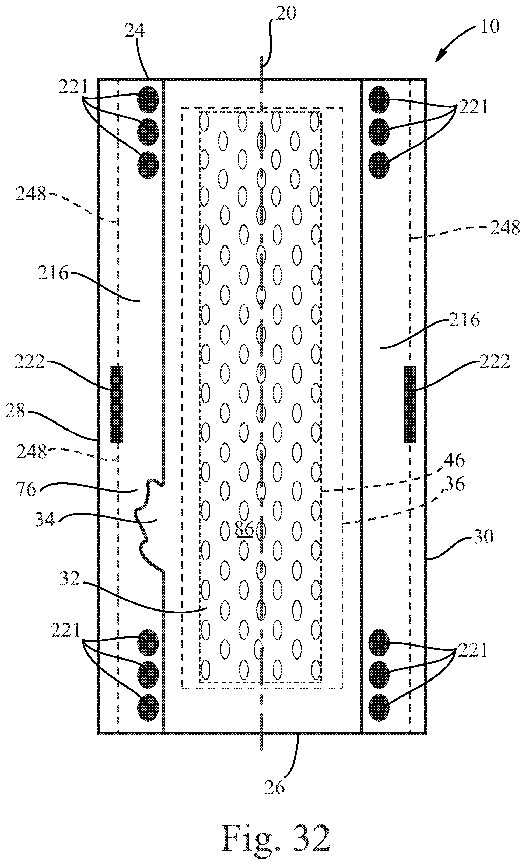

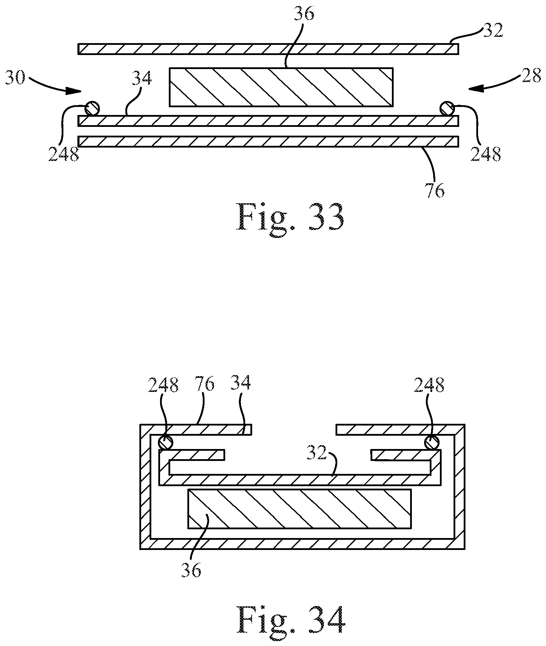

In some instances, discrete cuffs, like the pairs of cuffs 16 and 17 may not be provided in an absorbent pad. Instead, referring to Figs. 31-34, a pair of cuffs 216 may be integrally formed in the absorbent pad 10 by folding the first and second side edges 28 and 30 inwardly toward the central longitudinal axis 20. Fig. 31 is a perspective view photograph of an absorbent pad with folded over cuffs. Fig. 32 is a plan view of an absorbent pad 10 with folded over cuffs 216. Fig. 33 is an example cross-sectional view of the absorbent pad 10 prior to the cuffs 216 being folded toward the longitudinal axis 20. Fig. 34 is an example end view of the absorbent pad 10 of Fig. 32 before any tack down bonds are applied.

Referring to Figs. 32-34, the structure of the absorbent pad 10 is illustrated. The absorbent pad 10 comprises a topsheet 32, an absorbent core 36, a backsheet 34, and an outer cover nonwoven material 76. The absorbent core 36 is disposed at least partially between the topsheet 32 and the backsheet 34. Elastics 248 may also be positioned intermediate the topsheet 32 and the backsheet 34 or may be positioned intermediate the backsheet 34 and the outer cover nonwoven material 76. Portions of the topsheet 32, the backsheet 34, and the outer cover nonwoven material 76 may be folded towards the longitudinal axis 20 to create a structure like that illustrated in Figs. 31 and 32. The elastics 248 may only be joined to the cuff 216 in a joined area 222, much like joined area 122. The joined area 222 may comprise an adhesive that joins the elastics 248 to the topsheet 32 and/or backsheet 34 (or the backsheet 34 and/or the outer cover nonwoven material 76). One or more tack down bonds 221 (similar to tack down bonds 121 described herein) may also be present on the absorbent pad 10 proximate to the first end edge 24 and the second end edge 26. The tack down bonds 221 may be used to hold the ends of the cuffs 216 in the folded over configuration. In some instances, the elastics 248 may not be provided and the cuffs 216 may "stand" because of the tack down bonds 221. Any suitable number of tack down bonds 221 may be provided.

The tack down bonds 221 in combination with the joined area 222 of the elastics 248 may cause the cuffs 216 to "stand" more than without the elastics 248 and the joined area 222, as illustrated in Fig. 31. In some instances, the elastics 248 may be joined to the topsheet 32 and/or backsheet 34 (or to the backsheet 34 and/or the outer cover nonwoven material 76) along their full length or along most of their length. Other features, such as the wetness guards 12, 14, for example, of the absorbent pads 10 described herein may also be used with the absorbent pad of Figs. 30-34. One or more acquisition materials and/or distribution materials may also be provided at least partially intermediate the topsheet 32 and the absorbent core 36, similar to the acquisition material 46 described herein.

A total length of the absorbent pad 10, in a direction parallel with the central longitudinal axis of the absorbent pad, may be less than 500mm, less than 400mm, less than 300 mm, or less than 275mm, but at least 100mm. A total width of the absorbent pad, in a direction parallel with the central lateral axis, may be less than 150mm, less than 130 mm, less than 100mm, but at least 50mm.

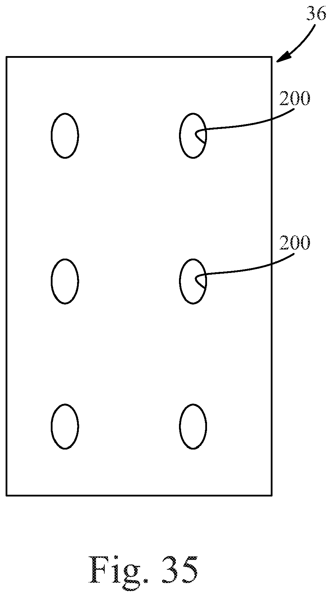

Referring to Fig. 35, an absorbent core 36 of any of the absorbent pads 10 disclosed herein may have apertures 200 defined therein. Any suitable number of apertures may be provided and the apertures 200 may have any suitable shape, such as round or ovate, for example. The apertures 200 may also have any suitable size. The apertures 200 may all be the same in size and/or shape or may be different in size and/or shape. The apertures 200 may be dispersed uniformly or non-uniformly in the absorbent core 36. The apertures 200 may make the absorbent core 36 more flexible and allow the absorbent core to fold or flex easier. The absorbent core may be shaped as discussed herein.

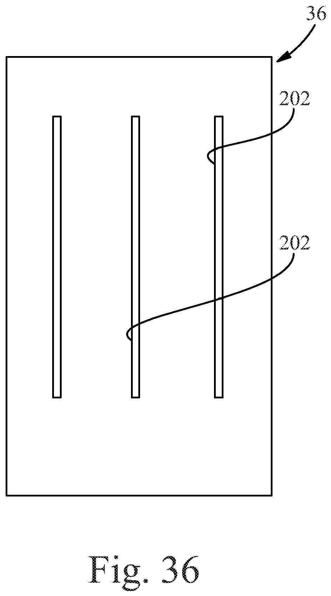

Referring to Fig. 36, an absorbent core 36 of any of the absorbent pads 10 disclosed herein may have slots 202 defined therein. Any suitable number of slots 202 may be provided and the slots 202. The slots 202 may all be the same in size and/or shape or may be different in size and/or shape. The slots 202 may be linear or non-linear, continuous or discontinuous. The slots 202 may extend in any suitable direction. The slots 202 may have any suitable width. In some instances, the slots 202 may not have material removed in their creation, but may only be cut lines in the absorbent core 36. The slots 202 may be distributed uniformly or non-uniformly in the absorbent core 36. The slots 202 may make the absorbent core 36 more flexible and allow the absorbent core 36 to fold or flex easier. The absorbent core may be shaped as discussed herein.

## Claims

1. An absorbent pad (10) configured to be positioned on a flat, horizontal surface to absorb bodily fluids exudates from babies positioned on the absorbent pad, the absorbent pad comprising:
a central longitudinal axis (20);
a central lateral axis (22);
a first end edge (24) on a first side of the central lateral axis;
a second end edge (26) on a second side of the central lateral axis;
a first side edge (28) on a first side of the central longitudinal axis;
a second side edge (30) on a second side of the central longitudinal axis;
a liquid permeable topsheet (32);
a liquid impermeable backsheet (34);
an absorbent core (36) disposed at least partially intermediate the liquid permeable topsheet and the liquid impermeable backsheet, wherein the absorbent core is rectangular;
a single pair of cuffs (16) extending at least partially intermediate the first end edge and the second end edge, wherein the cuffs each comprise an elastic (48), wherein each elastic is joined to the cuff only in a longitudinally central joined area, and wherein portions of the elastics outside of the longitudinally central joined areas are free from attachment to the cuffs; and
a first wetness guard (12) positioned on the first side of the central lateral axis and having a portion positioned proximate to the first end edge, wherein the first wetness guard is positioned over at least a first portion of the topsheet and crosses the central longitudinal axis, wherein the first wetness guard comprises a first nonwoven material (72) and a first liquid impermeable film (71), and wherein the first wetness guard forms a first portion of a wearer-facing surface of the absorbent pad;
a second wetness guard (14) positioned on the second side of the central longitudinal axis and having a portion positioned proximate to the second end edge, wherein the second wetness guard is positioned over at least a second portion of the topsheet and crosses the central longitudinal axis, wherein the second wetness guard comprises a second nonwoven material and a second liquid impermeable film, and wherein the second wetness guard forms a second portion of the wearer-facing surface;
wherein the first wetness guard overlaps a first portion of the absorbent core, and wherein the second wetness guard overlaps a second, different portion of the absorbent core; and
wherein the absorbent pad comprises a non-slip means on a surface of the absorbent pad opposite to the topsheet.

2. The absorbent pad according to Claim 1, wherein the first wetness guard has a first length in a direction parallel with the central longitudinal axis, wherein the second wetness guard has a second length in the direction parallel with the central longitudinal axis, and wherein the first length is different than the second length.

3. The absorbent pad according to Claim 1, wherein the first wetness guard has a first length in a direction parallel with the central longitudinal axis, wherein the second wetness guard has a second length in the direction parallel with the central longitudinal axis, and wherein the first length is substantially the same as the second length.

4. The absorbent pad according to any one of the preceding claims, comprising:
a garment-facing surface;
a first graphic (98) viewable from the garment-facing surface and on the first side of the central lateral axis; and
a second graphic (99) viewable from the garment-facing surface and on the second side;
wherein the first graphic is a mirror image of the second graphic relative to the central lateral axis to indicate reversibility to a caregiver.

5. The absorbent pad according to any one of the preceding claims, wherein a first portion of the first wetness guard is joined to the cuffs and/or the topsheet proximate to the first end edge of the absorbent pad, wherein a second portion of the first wetness guard is joined to the cuffs and/or the topsheet proximate to the first side edge, and wherein a third portion of the first wetness guard is joined to the cuffs and/or the topsheet proximate to the second side edge of the absorbent pad.

6. The absorbent pad according to any one of the preceding claims, wherein the first wetness guard has a first end positioned proximate to the first end edge of the absorbent pad, wherein the first wetness guard has a second end positioned intermediate the first end edge of the absorbent pad and the central lateral axis, and wherein the second end of the first wetness guard has at least a portion that is free of attachment with the topsheet.

7. The absorbent pad according to any one of Claims 1-6, wherein the second wetness guard has a first end positioned proximate to the first end edge of the absorbent pad, wherein the second wetness guard has a second end positioned intermediate the second end edge of the absorbent pad and the central lateral axis, and wherein the second end of the second wetness guard has at least a portion that is free of attachment with the topsheet.

8. The absorbent pad according to any one of the preceding claims, wherein the first wetness guard has a first width in a direction parallel to the central lateral axis, wherein the absorbent pad has a second width in the direction parallel to the central lateral axis, and wherein the first width is the same as or less than the second width.

9. The absorbent pad according to any one of the preceding claims, comprising an acquisition material positioned intermediate the absorbent core and the topsheet, wherein the topsheet is apertured, wherein the topsheet is hydrophobic, and wherein the first wetness guard is a discrete element that is joined to the cuffs and/or the topsheet.

10. The absorbent pad according to any one of the preceding claims, wherein each of the cuffs comprise three longitudinally extending fold lines, and wherein the longitudinally central joined areas are less than 4,45 cm.

11. The absorbent pad according to any one of the preceding claims, wherein the cuffs each comprise a free end and a terminal end, and wherein the terminal ends are positioned adjacent to or form a portion of the first side edge or the second side edge.

12. The absorbent pad according to any one of the preceding claims, wherein a total length of the absorbent pad, in a direction parallel with the central longitudinal axis, is less than 300 mm, and wherein a total width of the absorbent pad, in a direction parallel with the central lateral axis, is less than 130 mm.

13. A package comprising a plurality of the absorbent pads according to any one of the preceding claims, wherein the pads are not folded in the package.

## Patentansprüche

1. Absorptionseinlage (10), die dazu konfiguriert ist, auf einer ebenen, horizontalen Oberfläche angeordnet zu werden, um Körperflüssigkeitsausscheidungen von Babys, die auf der Absorptionseinlage angeordnet werden, zu absorbieren, wobei die Absorptionseinlage umfasst:
eine Mittellängsachse (20);
eine Mittelquerachse (22);
einen ersten Endrand (24) auf einer ersten Seite der Mittelquerachse;
einen zweiten Endrand (26) auf einer zweiten Seite der Mittelquerachse;
einen ersten Seitenrand (28) auf einer ersten Seite der Mittellängsachse;
einen zweiten Seitenrand (30) auf einer zweiten Seite der Mittellängsachse;
eine flüssigkeitsdurchlässige Oberschicht (32);
eine flüssigkeitsundurchlässige Unterschicht (34);
einen Absorptionskern (36), der wenigstens teilweise zwischen der flüssigkeitsdurchlässigen Oberschicht und der flüssigkeitsundurchlässigen Unterschicht angeordnet ist, wobei der Absorptionskern rechteckig ist;
ein einzelnes Paar von Bündchen (16), das sich wenigstens teilweise zwischen dem ersten Endrand und dem zweiten Endrand erstreckt, wobei die Bündchen jeweils ein Gummiband (48) umfassen, wobei jedes Gummiband mit dem Bündchen nur in einem in Längsrichtung mittleren gefalteten Bereich verbunden ist und wobei die Abschnitte der Gummibänder außerhalb der in Längsrichtung mittleren gefalteten Bereiche frei von einer Bindung an die Bündchen sind; und
einen ersten Feuchtigkeitsschutz (12), der auf der ersten Seite der Mittelquerachse angeordnet ist und einen Abschnitt aufweist, der dem ersten Endrand nahe gelegen angeordnet ist, wobei der erste Feuchtigkeitsschutz über wenigstens einem ersten Abschnitt der Oberschicht angeordnet ist und die Mittellängsachse kreuzt, wobei der erste Feuchtigkeitsschutz ein erstes Vliesmaterial (72) und eine erste flüssigkeitsundurchlässige Folie (71) umfasst und wobei der erste Feuchtigkeitsschutz einen ersten Abschnitt einer trägerseitigen Oberfläche der Absorptionseinlage bildet;
einen zweiten Feuchtigkeitsschutz (14), der auf der zweiten Seite der Mittellängsachse angeordnet ist und einen Abschnitt aufweist, der dem zweiten Endrand nahe gelegen angeordnet ist, wobei der zweite Feuchtigkeitsschutz über wenigstens einem zweiten Abschnitt der Oberschicht angeordnet ist und die Mittellängsachse kreuzt, wobei der zweite Feuchtigkeitsschutz ein zweites Vliesmaterial und eine zweite flüssigkeitsundurchlässige Folie umfasst und wobei der zweite Feuchtigkeitsschutz einen zweiten Abschnitt der trägerseitigen Oberfläche bildet;
wobei der erste Feuchtigkeitsschutz einen ersten Abschnitt des Absorptionskerns überlappt und wobei der zweite Feuchtigkeitsschutz einen zweiten, anderen Abschnitt des Absorptionskerns überlappt; und
wobei die Absorptionseinlage ein rutschhemmendes Mittel auf einer Oberfläche der Absorptionseinlage umfasst, die der Oberschicht entgegengesetzt ist.

2. Absorptionseinlage nach Anspruch 1, wobei der erste Feuchtigkeitsschutz eine erste Länge in einer Richtung aufweist, die parallel zu der Mittellängsachse verläuft, wobei der zweite Feuchtigkeitsschutz eine zweite Länge aufweist, die parallel zu der Mittellängsachse verläuft, und wobei die erste Länge anders als die zweite Länge ist.

3. Absorptionseinlage nach Anspruch 1, wobei der erste Feuchtigkeitsschutz eine erste Länge in einer Richtung aufweist, die parallel zu der Mittellängsachse verläuft, wobei der zweite Feuchtigkeitsschutz eine zweite Länge aufweist, die parallel zu der Mittellängsachse verläuft, und wobei die erste Länge im Wesentlichen gleich wie die zweite Länge ist.

4. Absorptionseinlage nach einem der vorstehenden Ansprüche, umfassend:
eine bekleidungsseitige Oberfläche;
eine erste Grafik (98), die von der bekleidungsseitigen Oberfläche und auf der ersten Seite der Mittelquerachse sichtbar ist; und
eine zweite Grafik (99), die von der bekleidungsseitigen Oberfläche und auf der zweiten Seite sichtbar ist;
wobei die erste Grafik ein Spiegelbild der zweiten Grafik in Bezug auf die Mittelquerachse ist, um einer Pflegeperson eine Umkehrbarkeit anzuzeigen.

5. Absorptionseinlage nach einem der vorstehenden Ansprüche, wobei ein erster Abschnitt des ersten Feuchtigkeitsschutzes mit den Bündchen und/oder der Oberschicht verbunden ist, die dem ersten Endrand der Absorptionseinlage nahe gelegen ist, wobei ein zweiter Abschnitt des ersten Feuchtigkeitsschutzes mit den Bündchen und/oder der Oberschicht verbunden ist, die dem ersten Seitenrand nahe gelegen sind, und wobei ein dritter Abschnitt des ersten Feuchtigkeitsschutzes mit den Bündchen und/oder der Oberschicht verbunden ist, die dem zweiten Seitenrand des Absorptionseinlage nahe gelegen sind.

6. Absorptionseinlage nach einem der vorstehenden Ansprüche, wobei der erste Feuchtigkeitsschutz ein erstes Ende aufweist, das dem ersten Endrand der Absorptionseinlage nahe gelegen angeordnet ist, wobei der erste Feuchtigkeitsschutz ein zweites Ende aufweist, das zwischen dem ersten Endrand der Absorptionseinlage und der Mittelquerachse angeordnet ist, und wobei das zweite Ende des ersten Feuchtigkeitsschutzes wenigstens einen Abschnitt aufweist, der frei von einer Bindung mit der Oberschicht ist.

7. Absorptionseinlage nach einem der Ansprüche 1 bis 6, wobei der zweite Feuchtigkeitsschutz ein erstes Ende aufweist, das dem ersten Endrand der Absorptionseinlage nahe gelegen angeordnet ist, wobei der zweite Feuchtigkeitsschutz ein zweites Ende aufweist, das zwischen dem zweiten Endrand der Absorptionseinlage und der Mittelquerachse angeordnet ist, und wobei das zweite Ende des zweiten Feuchtigkeitsschutzes wenigstens einen Abschnitt aufweist, der frei von einer Bindung mit der Oberschicht ist.

8. Absorptionseinlage nach einem der vorstehenden Ansprüche, wobei der erste Feuchtigkeitsschutz eine erste Breite in einer Richtung aufweist, die parallel zu der Mittelquerachse verläuft, wobei die Absorptionseinlage eine zweite Breite aufweist, die parallel zu der Mittelquerachse verläuft, und wobei die erste Breite das Gleiche wie oder weniger als die zweite Breite beträgt.

9. Absorptionseinlage nach einem der vorstehenden Ansprüche, umfassend ein Aufnahmematerial, das zwischen dem Absorptionskern und der Oberschicht angeordnet ist, wobei die Oberschicht durchlässig ist, wobei die Oberschicht hydrophob ist und wobei der erste Feuchtigkeitsschutz ein diskretes Element ist, das mit den Bündchen und/oder der Oberschicht verbunden ist.

10. Absorptionseinlage nach einem der vorstehenden Ansprüche, wobei jedes der Bündchen drei sich in Längsrichtung erstreckende Faltlinien umfasst und wobei die in Längsrichtung mittleren gefalteten Bereiche weniger als 4,45 cm messen.

11. Absorptionseinlage nach einem der vorstehenden Ansprüche, wobei die Bündchen jeweils ein freies Ende und ein endständiges Ende umfassen und wobei die endständigen Enden einem Abschnitt des ersten Seitenrandes oder des zweiten Seitenrandes benachbart angeordnet sind.

12. Absorptionseinlage nach einem der vorstehenden Ansprüche, wobei eine Gesamtlänge der Absorptionseinlage in einer Richtung, die parallel zu der Mittellängsachse verläuft, weniger als 300 mm beträgt und wobei eine Gesamtbreite der Absorptionseinlage in einer Richtung, die parallel zu der Mittelquerachse verläuft, weniger als 130 mm beträgt.

13. Verpackung, umfassend eine Vielzahl der Absorptionseinlagen nach einem der vorstehenden Ansprüche, wobei die Einlagen in der Verpackung nicht gefaltet sind.

## Revendications

1. Tampon absorbant (10) configuré pour être positionné sur une surface horizontale, plane, pour absorber des exsudats de fluides corporels de bébés positionnés sur le tampon absorbant, le tampon absorbant comprenant :
un axe longitudinal central (20) ;
un axe latéral central (22) ;
un premier bord d'extrémité (24) sur un premier côté de l'axe latéral central ;
un deuxième bord d'extrémité (26) sur un deuxième côté de l'axe latéral central ;
un premier bord latéral (28) sur un premier côté de l'axe longitudinal central ;
un deuxième bord latéral (30) sur un deuxième côté de l'axe longitudinal central ;
une feuille de dessus perméable aux liquides (32) ;
une feuille de fond imperméable aux liquides (34) ;
une âme absorbante (36) disposée au moins partiellement entre la feuille de dessus perméable aux liquides et la feuille de fond imperméable aux liquides, dans lequel l'âme absorbante est rectangulaire ;
une seule paire de revers (16) s'étendant au moins partiellement entre le premier bord d'extrémité et le deuxième bord d'extrémité, dans lequel les revers comprennent chacun un élastique (48), dans lequel chaque élastique est relié au revers uniquement dans une zone de liaison longitudinalement centrale, et dans lequel des parties des élastiques à l'extérieur des zones de liaison longitudinalement centrales sont exemptes d'attache sur les revers ; et
une première protection contre l'humidité (12) positionnée sur le premier côté de l'axe latéral central et ayant une partie positionnée à proximité du premier bord d'extrémité, dans lequel la première protection contre l'humidité est positionnée sur au moins une première partie de la feuille de dessus et traverse l'axe longitudinal central, dans lequel la première protection contre l'humidité comprend un premier matériau non tissé (72) et un premier film imperméable aux liquides (71), et dans lequel la première protection contre l'humidité forme une première partie d'une surface tournée vers le porteur du tampon absorbant ;
une deuxième protection contre l'humidité (14) positionnée sur le deuxième côté de l'axe longitudinal central et ayant une partie positionnée à proximité du deuxième bord d'extrémité, dans lequel la deuxième protection contre l'humidité est positionnée sur au moins une deuxième partie de la feuille de dessus et traverse l'axe longitudinal central, dans lequel la deuxième protection contre l'humidité comprend un deuxième matériau non tissé et un deuxième film imperméable aux liquides, et dans lequel la deuxième protection contre l'humidité forme une deuxième partie de la surface tournée vers le porteur ;
dans lequel la première protection contre l'humidité chevauche une première partie de l'âme absorbante, et dans lequel la deuxième protection contre l'humidité chevauche une deuxième partie, différente, de l'âme absorbante ; et
le tampon absorbant comprenant un moyen antidérapant sur une surface du tampon absorbant opposée à la feuille de dessus.

2. Tampon absorbant selon la revendication 1, dans lequel la première protection contre l'humidité a une première longueur dans une direction parallèle à l'axe longitudinal central, dans lequel la deuxième protection contre l'humidité a une deuxième longueur dans la direction parallèle à l'axe longitudinal central, et dans lequel la première longueur est différente de la deuxième longueur.

3. Tampon absorbant selon la revendication 1, dans lequel la première protection contre l'humidité a une première longueur dans une direction parallèle à l'axe longitudinal central, dans lequel la deuxième protection contre l'humidité a une deuxième longueur dans la direction parallèle à l'axe longitudinal central, et dans lequel la première longueur est sensiblement la même que la deuxième longueur.

4. Tampon absorbant selon l'une quelconque des revendications précédentes, comprenant :
une surface tournée vers le vêtement ;
un premier graphisme (98) pouvant être observé depuis la surface tournée vers le vêtement et sur le premier côté de l'axe latéral central ; et
un deuxième graphisme (99) pouvant être observé depuis la surface tournée vers le vêtement et sur le deuxième côté ;
dans lequel le premier graphisme est une image miroir du deuxième graphisme par rapport à l'axe latéral central pour indiquer la réversibilité à un prestataire de soins.

5. Tampon absorbant selon l'une quelconque des revendications précédentes, dans lequel une première partie de la première protection contre l'humidité est reliée aux revers et/ou à la feuille de dessus à proximité du premier bord d'extrémité du tampon absorbant, dans lequel une deuxième partie de la première protection contre l'humidité est reliée aux revers et/ou à la feuille de dessus à proximité du premier bord latéral, et dans lequel une troisième partie de la première protection contre l'humidité est reliée aux revers et/ou à la feuille de dessus à proximité du deuxième bord latéral du tampon absorbant.

6. Tampon absorbant selon l'une quelconque des revendications précédentes, dans lequel la première protection contre l'humidité a une première extrémité positionnée à proximité du premier bord d'extrémité du tampon absorbant, dans lequel la première protection contre l'humidité a une deuxième extrémité positionnée entre le premier bord d'extrémité du tampon absorbant et l'axe latéral central, et dans lequel la deuxième extrémité de la première protection contre l'humidité a au moins une partie qui est exempte d'attache avec la feuille de dessus.

7. Tampon absorbant selon l'une quelconque des revendications 1 à 6, dans lequel la deuxième protection contre l'humidité a une première extrémité positionnée à proximité du premier bord d'extrémité du tampon absorbant, dans lequel la deuxième protection contre l'humidité a une deuxième extrémité positionnée entre le deuxième bord d'extrémité du tampon absorbant et l'axe latéral central, et dans lequel la deuxième extrémité de la deuxième protection contre l'humidité a au moins une partie qui est exempte d'attache avec la feuille de dessus.

8. Tampon absorbant selon l'une quelconque des revendications précédentes, dans lequel la première protection contre l'humidité a une première largeur dans une direction parallèle à l'axe latéral central, le tampon absorbant ayant une deuxième largeur dans la direction parallèle à l'axe latéral central, et dans lequel la première largeur est identique ou inférieure à la deuxième largeur.

9. Tampon absorbant selon l'une quelconque des revendications précédentes, comprenant un matériau d'acquisition positionné entre l'âme absorbante et la feuille de dessus, dans lequel la feuille de dessus est perforée, dans lequel la feuille de dessus est hydrophobe, et dans lequel la première protection contre l'humidité est un élément discret qui est relié aux revers et/ou à la feuille de dessus.

10. Tampon absorbant selon l'une quelconque des revendications précédentes, dans lequel chacun des revers comprend trois lignes de pliage s'étendant longitudinalement, et dans lequel les zones de liaison longitudinalement centrales sont inférieures à 4,45 cm.

11. Tampon absorbant selon l'une quelconque des revendications précédentes, dans lequel les revers comprennent chacun une extrémité libre et une extrémité terminale, et dans lequel les extrémités terminales sont positionnées adjacentes à ou forment une partie du premier bord latéral ou du deuxième bord latéral.

12. Tampon absorbant selon l'une quelconque des revendications précédentes, dans lequel une longueur totale du tampon absorbant, dans une direction parallèle à l'axe longitudinal central, est inférieure à 300 mm, et dans lequel une largeur totale du tampon absorbant, dans une direction parallèle à l'axe latéral central, est inférieure à 130 mm.

13. Conditionnement comprenant une pluralité des tampons absorbants selon l'une quelconque des revendications précédentes, dans lequel les tampons ne sont pas pliés dans le conditionnement.
